# EUROPEAN PATENT APPLICATION

(11) **EP 1 186 305 A1**
(43) Date of publication of application: **13.03.2002**
(21) Application number: 00250298.7
(22) Date of filing: 07.09.2000
(51) Int. Cl.: A61K 49/04, C07C 235/16, C07C 311/09, C07C 237/46, C07C 233/65, C07C 233/69

(54) **New brominated compounds as contrast media for X-ray mammography**

(71) Applicant: Schering Aktiengesellschaft, 13353 Berlin (DE)
(72) Inventor: González Tavares, Leonor, 28029 Madrid (ES); Carretero Col n, José M., 28030 Madrid (ES); Harto Martinez, Juan R., 28005 Madrid (ES); Martin Jiménez, José L., 28220 Majadahonda (Madrid) (ES); Martinez Sanz, Antonio, 28047 Madrid (ES); Riefke, Björn, 28224 Pozuelo de Alarcon (Madrid) (ES); Gries, Heinz, 10171 Berlin (DE)

(57) **Abstract**

The invention regards new brominated compounds containing one or two phenyl rings with favorable physico-chemical properties, good tolerance profile, stability and suitable pharmacokinetic profile as contrast media for X-ray mammography, their pharmaceutical formulations and radiological application. The described new bromine contrast media will increase the sensitivity and specificity of X-ray mammography especially in problematic cases. The new contrast media have a higher X-ray absorption than iodine in the energy range used by mammography, a high hydrophilicity, lower toxicity and better tolerance profile than corresponding iodinated compounds.

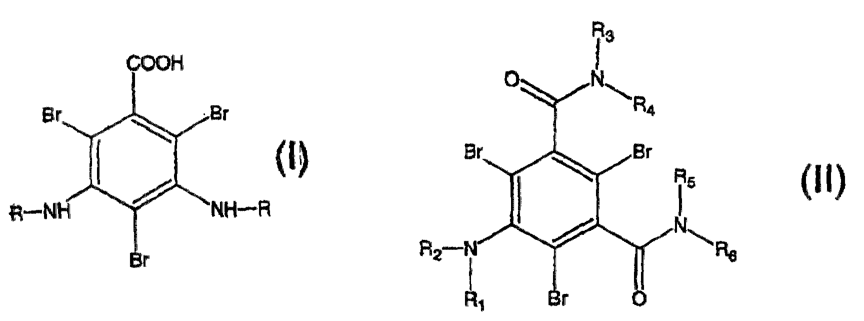

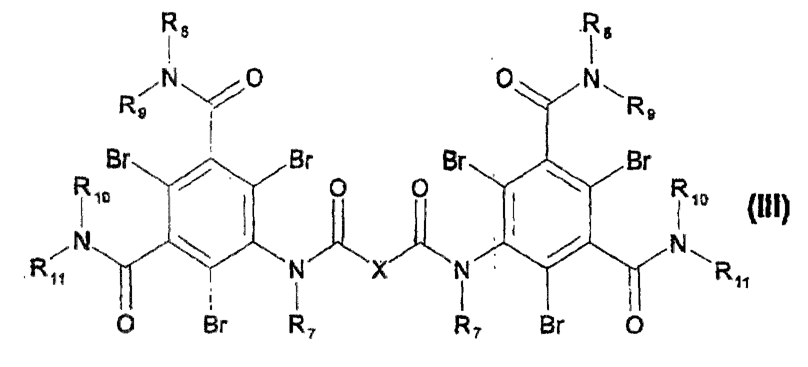

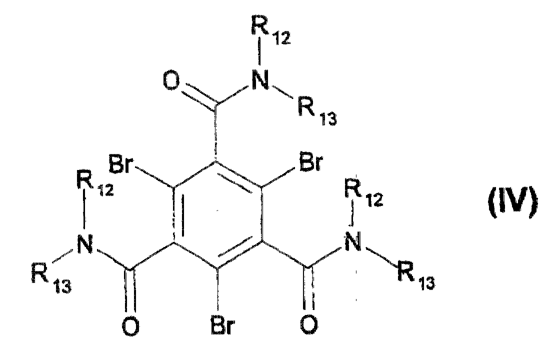

## Description

The invention regards new brominated compounds with favorable physico-chemical properties, good tolerance profile, stability and suitable pharmacokinetic profile as contrast media for X-ray mammography, their pharmaceutical formulations and radiological application. The described new bromine contrast media will increase the sensitivity and specificity of X-ray mammography especially in problematic cases. The new contrast media have a higher X-ray absorption than iodine in the energy range used by mammography, a high hydrophilicity, lower toxicity and better tolerance profile than corresponding iodinated compounds.

### Introduction

Mammography is today the most effective single imaging modality for the detection of breast cancer. In mammography breast tissue is exposed to X-ray radiation of low energies between 20 and 30 kV, where contrast between glandular tissue, fat, water and microcalcifications yields highest values at an acceptable radiation dose. Mammographic images have a very high spatial resolution of about 200 µm, necessary for the detection of microcalcifications as an important sign for early breast cancer. During the past decades mammography has evolved to an optimized procedure with wide distribution of high quality standards. Because of the good diagnostic-value to cost relation mammography is applied in screening programs for breast cancer. Sensitivity and specificity are strongly age dependent and decreasing when younger women were examined. A problem which is associated to the higher density of glandular tissue in younger woman. Recently the increasing occurrence of dense breasts in older women was associated to the more widespread application of hormone replacement therapy. It was estimated that problematic cases contribute to 30% of evaluated patients in mammography. Additionally 60 to 80% of biopsies directed by mammography are benign. This means a very high rate of unnecessary interventions, scar formation and frightened patients.

Dedicated contrast media for mammography may be used to improve the sensitivity and specificity of this widely distributed and accepted technique for breast imaging especially in problematic mammographic cases in the future. The pattern of contrast media enhancement would serve as a physiological parameter added to the morphological image indicative for tumor angiogenesis and grade of malignancies (Jules K.A.: Tumor angiogenesis and its relation to contrast enhancement on computer tomography: a review. European Journal of Radiology 30, 198-205, 1990).

In MRI the non-specific hydrophilic low molecular contrast agent Gd-DTPA (Magnevist® ) has been proven to be very helpful and valuable in the detection and characterization of breast lesion (Heywang S.H., Hahn D., Schmidt H., Eiermann W. Bassermann R., Lissner J.: MR imaging of the breast using Gd-DTPA. J Comp Assist Tomogr 10, 199-204, 1986) Also in CT-imaging iodinated contrast media were used with high sensitivity and specificity (Teifke A., Schweden F., Cagil H., Kauczor H.U., Mohr W. Thelen M.: Spiral-Computer Tomographie der Mamma. Fortschr Röntgenstr 161, 495-500, 1994). Despite their excellent clinical results both techniques are not widely accepted because of high costs and time consuming procedure in MRI and application of a relative high radiation dose to the patient with CT. In projection imaging of the breast contrast media were applied in DSA-systems for the detection of breast lesions in the past (Fuchs H.D., Stigl R.: Diagnose und Differentioaldiagnose des Mammakarzinoms mittels intravenöser DSA. Fortschr Röntgenstr 142 (3), 314-320, 1985) These applications were not followed in the past despite good imaging results but because lack of spatial resolution and poor contrast sensitivity. In the galactography procedure contrast media were directly injected in the ductal ducts for the detection and characterization of lesions. In contrast intravenous injection of contrast media are by far less invasive.

Usually mammography is performed at energies lower than 30 kV, this means values lower than the k-edge for iodine (33 keV). In this energy range atoms like bromine, gadolinium, tungsten and bismuth have higher absorption than iodine. This was pointed out by Speck (EP 0 885 616 A1). Surprisingly conventional film based mammography and the newly developed digital mammography have the sensitivity to detect contrast material down to concentrations of 0.5 mg I/ml or molar concentration of other elements equivalent to this concentration (see example 27a). These values are indicating contrast sensitivity of mammography in the same range as reported for CT.

In earlier patent applications (FR 2736051-A1, EP 073715 A1, EP 074307 A1, EP 074 309 A1, EP 0 118 348 A1, FR 2541272-A1) described substances containing bromine, are claimed only for the application in conventional X-ray techniques not for X-ray mammography.

We have been able to demonstrate the usefulness and advantages of newly synthesized benzoic acid derivatives, containing bromine for X-ray mammography.

Moreover, because the lower atomic diameter of bromine, a brominated chemical structure presents higher hydrophilicity, better tolerance profile, lower toxicity, a better stability and lower production costs than the corresponding iodinated compounds.

According to this invention we have demonstrated new chemical structures of benzene derivatives containing bromine which fulfil the requirements for the application as contrast media for mammography: maximum opacification, chemical stability, higher hydrophilicity, lower viscosity, better tolerance, minimal osmotic effects (mainly in the case of dimeric compounds), and a favorable pharmacokinetic profile.

In further accordance with the present invention, radiological composition may be prepared containing one of the aforementioned compounds of the invention as an X-ray contrast agent together with a pharmaceutically acceptable radiological vehicle. Pharmaceutically acceptable vehicles include those that are suitable for injection such as aqueous buffer solutions; e.g. tris-(hydroxymethyl)aminomethane and its salts , phosphate, citrate, bicarbonate, etc., sterile water for injection, physiological saline and balanced ionic solutions containing chloride and/or bicarbonate salts of normal blood plasma cations such as Ca, Na, K and Mg. The vehicles may contain a chelating amount, e.g., a small amount of ethylenediamine tetraacetic acid, the calcium disodium salt, or other pharmaceutically acceptable chelating agent.

The concentration of the contrast agent varies with the field of use. For mammography purposes the concentration of bromine is generally 10-400 mg/mL and the dose 20 to 500 mL.

According to the previous experiments, the invention relates to X-ray contrast agents, more particularly, to novel brominated X-ray contrast agents.

This invention refers to the monomeric ionic derivatives of 3,5-diamino-2,4,6-tribromobenzoic acid, having the following structure: 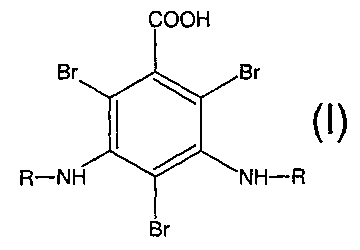 in which:
R is -COCH₂OH, -COCH₂OCH₃, -SO₂CH₂Br or - COCH₃

Preferred compounds of formula **I** are those in which
R is -COCH₂OH, -COCH₂OCH₃ or -SO₂CH₂Br

### (Examples 1, 2 and 3, respectively)

The present invention is also directed to new monomeric nonionic derivatives of tribromoisophthalamide, having the following structure: 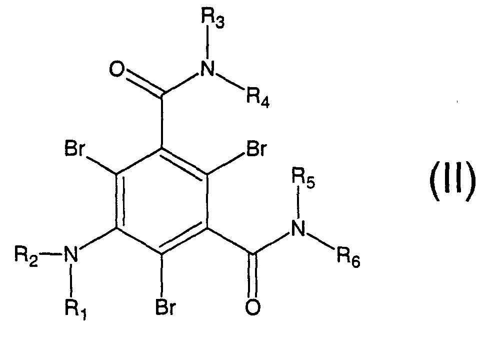 in which the substituents R₁, R₂, R₃, R₄, R₅, and R₆ are given in table 1

**Table 1.**

| Compounds of formula **II** | | | | | |
|---|---|---|---|---|---|
| **R**_{**1**} | **R**_{**2**} | **R**_{**3**} | **R**_{**4**} | **R**_{**5**} | **R**_{**6**} |
| H | COCH₂OH | CH₃ | CH₂CHOHCH₂OH | H | CH₂CHOHCH₂OH |
| H | COCH₂OH | H | CH₂CHOHCH₂OH | H | CH₂CHOHCH₂OH |
| H | COCH₂OH | H | CH₂CHOHCH₂OH | H | CH₂CH₂OH |
| CH₃ | COCH₂OH | H | CH₂CHOHCH₂OH | H | CH₂CHOHCH₂OH |
| CH₂CH₂OH | COCH₂OH | H | CH₂CHOHCH₂OH | H | CH₂CHOHCH₂OH |
| CH₂CHOHCH₂OH | COCH₂OH | H | CH₂CHOHCH₂OH | H | CH₂CHOHCH₂OH |
| H | SO₂CH₂Br | CH₃ | CH₂CHOHCH₂OH | H | CH₂CHOHCH₂OH |
| H | COCH₂OH | CH₃ | CH₂CHOHCH₂OH | H | H |
| H | COCH₂OH | H | CH(CH₂OH)CHOHCH₂OH | H | H |
| H | COCH₂OH | CH₂CH₂OH | CH₂CH₂OH | CH₂CH₂OH | CH₂CH₂OH |
| CH₂CH₂OH | COCH₂OH | H | CH(CH₂OH)CHOHCH₂OH | H | H |
| H | COCH₂OH | CH₃ | CH₂CHOHCH₂OH | CH₃ | CH₂CHOHCH₂OH |
| H | COCH₂OCH₃ | CH₃ | CH₂CHOHCH₂OH | CH₃ | CH₂CHOHCH₂OH |
| H | COCH₂OH | H | CH₂CHOHCH₂OH | H | CH(CH₂OH)₂ |
| CH₂CH₂OH | COCH₃ | H | CH(CH₂OH)₂ | H | H |
| CH₂CH₂OH | COCH₃ | H | CH(CH₂OH)CHOHCH₂OH | H | H |

The present invention is further directed to new symmetrical dimeric nonionic derivatives of tribromoisophthalamide connected at the 5-N position by means of a diamide, having the general formula III: 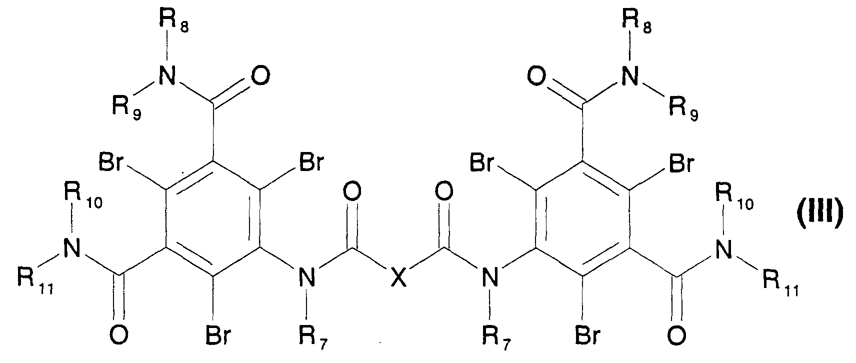 wherein
- R₇: is hydrogen, straight-chain or branched C₁-C₄-alkyl or mono-or polyhydroxyalkyl,
- R₈: is hydrogen, straight-chain or branched C₁-C₄-alkyl or mono-or polyhydroxyalkyl,
- R₉: is mono-or polyhydroxyalkyl,
- R₁₀: is hydrogen, straight-chain or branched C₁-C₄-alkyl or mono-or polyhydroxyalkyl,
- R₁₁: is hydrogen, straight-chain or branched C₁-C₄-alkyl or mono-or polyhydroxyalkyl,
- X: is straight-chain or branched C₁-C₄-alkyl or C₁-C₄-alkoxy-C₁-C₄-alkyl

The present invention is further directed to new monomeric derivatives of 2.4.6-Tribromobenzene-1.3.5-tricarboxylic acid (Tribromo-trimesic acid), having the structure IV 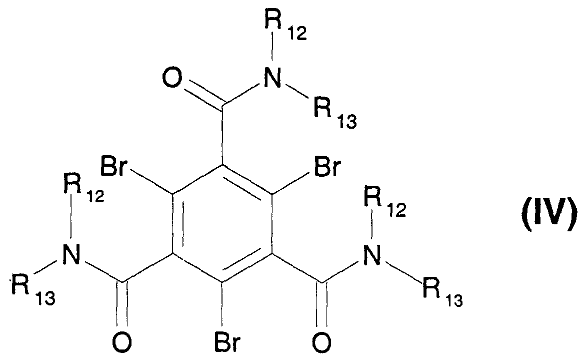 wherein
- R₁₂: is hydrogen, straight-chain or branched C₁-C₄-alkyl or mono-or polyhydroxy-C₁-C₄-alkyl,
- R₁₃: is straight-chain or branched C₁-C₄-alkyl or mono-or polyhydroxy-C₁- C₄-alkyl,

All lower alkyl residues defined above contain 1-4 carbon atoms. Preferred examples are methyl, ethyl and propyl. Methyl is most preferred. They may be straight-chain or branched.
The alkyl residue in the mono-or polyhydroxyalkyl residues can be straight-chain or branched. Preferably suitable are alkyl residues of 2-5, preferably 2-3 carbon atoms. The hydroxy groups in the alkyl residue can be present as primary and/or secondary and/or tertiary hydroxy groups. The alkyl groups can contain 1-5, preferably 1-3 hydroxy groups. Examples include tris (hydroxy methyl) methyl, hydroxyethyl, especially 1,3- and 2,3-dihydroxypropyl, 2,3-dihydroxy 1-hydroxymethylpropyl and 1,3,4-trihydroxy-threo-but-2-yl.
The present invention comprises monionic X-ray contrast media which are distinguished by a high chemical stability, especially also under the conditions of heat sterilisation.

### Examples

The following examples should illustrate the invention without limitation on presented examples.

### Example 1

### Synthesis of 2,4,6-tribromo-3,5-dihydroxyacetylaminobenzoic acid

### a) 3,5-Diamino-2,4,6-tribromobenzoic acid

To a suspension of 3,5-diaminobenzoic acid (30.2 g, 0.2 moles) in water (1.2 L) was added sufficient concentrated hydrochloric acid to complete solution (approximately pH=1). Bromine (33.8 mL, 0.65 moles) was added over one hour and the mixture reaction stirred at room temperature for two hours.

The pH was adjusted to approximately 6 with 50% sodium hydroxide (115 ml) and the reaction mixture treated with sodium hydrosulfite (7 g) to decolour the resulting solution.

Sufficient concentrated hydrochloric acid was added to adjust pH to 1 and the precipitate was filtered, washed with water and dried in vacuo to obtain the product, 3,5-diamino-2,4,6-tribromobenzoic acid (58.2 g, 75.6 %). The product was used in the next step without further purification.
- ¹H-NMR (200 MHz, DMSO-d₆):: δ= 4.9 (broad signal, 4H, exchangeable with D₂O, NH), 12.15 (broad signal, 1H, exchangeable with D₂O, COOH).
- ¹³C-NMR (200 MHz, DMSO-d₆):: δ= 168.2 (CO), 144.6, 139.4, 103.1 (C-Br), 102.2 (C-Br).

### b) 3, 5-Diacetoxyacetylamino-2,4,6-tribromobenzoic acid

To an ice-cold solution of 3,5-diamino-2,4,6-tribromobenzoic acid **(example 1a)** (45 g, 0.115 moles) in DMA (135 mL) was added distilled acetoxyacetyl chloride (78.5 g, 0.575 moles) over a thirty-minute period. The mixture was removed from the ice bath and stirred at room temperature for 16 hours.

The product was isolated by pouring the reaction mixture into ice water (1.5 L). The precipitate was filtered, washed with water and dried in vacuo to yield 3,5-diacetoxyacetylamino-2, 4, 6-tribromobenzoic acid (62.3 g, 92.0 %). The product was used in the next step without further purification.
- ¹H-NMR (200 MHz, DMSO-d₆):: δ= 12.2 (broad signal, 1H, exchangeable with D₂O, CO₂H), 9.9 (2s, 2H, exchangeable with D₂O, NH), 4.61 (s, 4H, COCH₂OAc), 2.2 (s, 6H, COCH₃).
- ¹³C-NMR (200 MHz, DMSO-d₆):: δ= 169.2 (CO), 168.4 (CO), 168.2 (CO), 139.4, 135.3, 113.5 (C-Br), 108.3(C-Br), 62.1 (CH₂), 20.5 (CH₃).

### c) 2,4,6-Tribromo-3,5-dihydroxyacetylaminobenzoic acid

To a suspension of 3,5-diacetoxyacetylamino-2,4,6-tribromobenzoic acid **(example 1b)** (60 g, 0.101 moles) was added sufficient 20% sodium hydroxide to adjust the pH at 11. The solution was stirred two hours at room temperature and then acidified to pH 1 with concentrated hydrochloric acid to precipitate 2,4,6-tribromo-3,5-dihydroxyacetylaminobenzoic acid . The precipitate was filtered off and washed with water.

The collected precipitate was redissolved in water (600 mL) plus sufficient 20 % sodium hydroxide to complete solution. The pH was adjusted to approximately 5 with acetic acid and the solution was treated with decolorizing charcoal (6 g) 1 hour at room temperature. The charcoal was filtered off and the solution was slowly added to a hot (70°C), stirred solution of hydrochloric acid (27 mL concentrated acid diluted with 54 mL water). The mixture was chilled and the product collected, washed and dried in vacuo at 60°C. This treatment was repeated once.

The product was further purified by once precipitating the acid from a solution of the sodium salt in water to yield 2,4,6-tribromo-3,5-dihydroxyacetylaminobenzoic acid (42 g, 82.3 %).
- ¹H-NMR (200 MHz, DMSO-d₆):: δ = 11.3 (broad signal, 1H, exchangeable with D₂O, CO₂H and OH), 9.8 (2s, 2H, exchangeable with D₂O, NH), 4.01 (s, 4H, COCH₂).
- ¹³C-NMR (200 MHz, DMSO-d₆):: δ = 168.9 (CO), 168.2 (CO), 137.8, 134.2, 113.3 (C-Br), 107.9 (C-Br), 61.6 (CH₂).

### Example 2

### Synthesis of 2,4,6-tribromo-3,5-dimethoxyacetylaminobenzoic acid

A solution of 3,5-diamino-2,4,6-tribromobenzoic acid **(example 1a)** (75 g, 0.192 moles) in DMA (150 mL) was cooled to 5° C: Methoxyacetylchloride (104.1 g, 0.96 moles) was added slowly keeping the temperature at 5-10°C. When the addition was complete, the reaction mixture was allowed to warm to room temperature and was stirred for 24 hours.

The reaction mixture was poured into an ice water slurry (1.6 L) with vigorous stirring. The precipitate was filtered off and washed with water.

The collected precipitate was suspended in water (750 mL) and sufficient 20% sodium hydroxide added to complete solution. The pH was adjusted to approximately 5 with acetic acid and the solution was treated with decolourising charcoal (7.5 g) 1 hour at room temperature. The charcoal was filtered off and the solution was slowly added to a hot (70°C), stirred solution of hydrochloric acid (37 mL concentrated acid diluted with 74 mL water). The mixture was chilled and the product collected, washed and dried in vacuo at 60°C. This treatment was repeated once.

The product was further purified by once precipitating the acid from a solution of the sodium salt in water to yield 2,4,6-tribromo-3,5-dimethoxyacetylbenzoic acid (83.5 g, 81.6 %).
- ¹H-NMR (200 MHz, DMSO-d₆):: δ= 12.8 (broad signal, 1H, exchangeable with D₂O, CO₂H), 9.8 (2s, 2H, exchangeable with D₂O, NH), 4.2 (s, 4H, CH₂), 3.5 (s, 6H, CH₃).
- ¹³C-NMR (200 MHz, DMSO-d₆):: δ= 169.2 (CO), 168.1 (CO), 138.0, 133.6, 112.8 (C-Br), 106.9 (C-Br), 71.1 (CH₂), 59.3 (CH₃).

### Example 3

### Synthesis of 2,4,6-tribromo-3,5-dibromomethanesulfonylamino benzoic acid

To a solution of 3,5-diamino-2,4,6-tribromobenzoic acid **(example 1a)** (55 g, 0.14 moles) in DMA (165 mL) was added distilled bromomethylsulfonyl bromide (166.48 g, 0.70 moles) (*J*. *Am. Chem. Soc*. **1986,** *108(15),* 4568-80) and the reaction mixture was heated at 60 °C for 16 hours.

The solid product was isolated by pouring the reaction mixture into ice water (1.5 L). The precipitate was filtered, washed with water and dried.

The collected precipitate was suspended in water (550 mL) and sufficient 20% sodium hydroxide added to complete solution. The pH was adjusted to approximately 5 with acetic acid and the solution was treated with decolorizing charcoal (5.5 g) 1 hour at room temperature. The charcoal was filtered off and the solution was slowly added to a hot (70°C), stirred solution of hydrochloric acid ( 25 mL concentrated acid diluted with 50 mL water). The mixture was chilled and the product collected, washed and dried in vacuo at 60°C. This treatment was repeated once.

The product was further purified by once precipitating the acid from a solution of the sodium salt in water to yield 2,4,6-tribromo-3,5-dibromomethylsulfonylaminobenzoic acid (76 g, 76.6 %).
- ¹H-NMR (200 MHz, DMSO-d₆):: δ= 14.8 (broad signal, 1H, exchangeable with D₂O, CO₂H), 9.9 (2s, 2H, exchangeable with D₂O, NH), 5.3 (s, 4H, CH₂).
- ¹³C-NMR (200 MHz, DMSO-d₆):: δ= 168.1 (CO), 144.4, 137.64, 108.08 (C-Br), 106.6 (C-Br), 47.3 (CH₂).

### Example 4

### Synthesis of 2,4,6-tribromo-5-hydroxyacetylamino-N,N'-bis-(2,3-dihydroxypropyl)-N-methylisophthalamide

### a) 5-Nitro-N-(2,3-dihydroxypropyl)isophthalic acid monoamide

To a solution of 5-nitroisophthalic acid monomethyl ester (22.5 g, 0.1 moles) in methanol (100 mL), triethylamine (10.1 g, 0.1 moles) and 3-amino-1,2-propanediol (10.02 g, 0.11 moles) were added with stirring and heated at solvent reflux for three hours. The solution was evaporated to dryness under reduced pressure. The residue was treated with cold water, filtered, and washed with cold water. The white solid was dried in vacuo to obtain 5-nitro-N-(2,3-dihydroxypropyl)isophthalic acid monoamide (26.9 g, 95%). The product was used in the next step without purification.
- ¹H-NMR (200MHz, DMSO-d₆):: δ= 3.20-3.40 (m, 4H, two methylene units), 4.73 (m, 3H, 2OH and CH)), 6.92 (bs, 1H, NH), 8.68 (s, 1H aromatic), 8.87 (s, 1H aromatic), 9.02 (s, 1H aromatic), and 9.08 (m, 1H, NH).
- ¹³C-NMR (200MHz, DMSO-d₆):: δ=43.50 (NH-CH₂), 64.15 (CH₂OH), 70.55 (CH), 126.13/133.01/134.04 (aromatic CH), 136.54 (C-COOH),136.60 (C-CONH), 148.18 (C-NO₂), 164.20 (CO in CONH), and 165.40 (CO in COOH).

### b) 5-Amino-N-(2,3-dihydroxypropyl)isophthalic acid monoamide

To a suspension of 5-nitro-N-(2,3-dihydroxypropyl)isophthalic acid monoamide (25 g, 0.09 moles) (**example 4a**) in water (100 mL) at 50°C., 25% ammonium hydroxide (5.5 mL) was added dropwise (the pH increased from 1.69 to 3.55). The mixture was diluted with water (100 mL) and hydrogenated with 1.5 g of palladium over charcoal at 70°C for three hours. The reaction medium was cooled at room temperature and filtered off. Hydrochloric acid was added (pH=2.5) and the solution was evaporated to dryness under reduced pressure. The residue was treated with methanol (60 mL), filtered off, and dried at 50°C in vacuo to obtain 5-amino-N-(2,3-dihydroxypropyl)isophthalic acid monoamide (20.8 g, 93%).
- ¹H-NMR (200MHz, DMSO-d₆):: δ=3.20-3.50 (m, 4H, two methylene units), 3.34 (m, 2H, two hydroxyl groups), 3.65(m, 1H, CH), 6.68 (bs, 2H, NH₂), 7.75 (m/m, 2H aromatic), 8.20 (s, 1H aromatic), and 8.75 (m, 1H, NH).
- ¹³C-NMR (200MHz, DMSO-d₆):: δ=43.53 (NH-CH₂), 64.18 (CH₂-OH), 70.53 (CH), 126.25 (aromatic C4), 126.36 (aromatic C6), 126.96 (aromatic C2), 132.85 (C-COOH), 134.16 (C-CONH), 136.85 (C-NO₂), 165.66 (CO in CONH), and 166.42 (CO in COOH).

### c) 5-Amino-2,4,6-tribromo-N-(2,3-dihydroxypropyl)isophthalic acid monoamide

To a solution of 5-amino-N-(2,3-dihydroxypropyl)isophthalic acid monoamide (25g, 0.1 moles) **(example 4b)** in water (1L), bromine (16.7 mL, 0.325 moles) was added dropwise with stirring at room temperature in the course of half hour.

The solution was stirred an additional hour and pH adjusted to 9 with 20% aqueous sodium hydroxide. The pH was decreased to 3.8 with sodium hydrosulphite. This operation was repeated until clear reaction medium. It was evaporated to dryness under reduced pressure. The residue was treated with methanol (100 mL) and filtered. The filtrate was evaporated to dryness under reduced pressure. The residue was treated with isopropanol (100 mL) and filtered. The solid was dried in vacuo at room temperature to obtain 5-amino-2,4,6-tribromo-N-(2,3-dihydroxypropyl)isophthalic acid monoamide (47 g, 96%).
- ¹H-NMR (200MHz, DMSO-d₆):: δ=3.00-3.50 (m, 4H, two methylene units), 3.65 (m, 1H, CH), 4.60 (m, 2H, NH₂) 4.80 (m, 2H, two hydroxyl groups), and 8.38 ppm (m, 1H, NH).
- ¹³C-NMR (200MHz, DMSO-d₆):: δ=42.39 (NH-CH₂), 63.83 (CH₂OH), 70.28 (CH), 100.58/101/05/103.58 (C-Br), 139.82 (C-COOH), 142.04 (C-CONH), 147.63 (C-NH₂), 166.58 (CO in CONH), and 168.13 (CO in COOH).

### d) 5-Amino-2,4,6-tribromo-N-(2,3-diacetoxypropyl)isophthalic acid monoamide

To a solution of 5-amino-2,4,6-tribromo-N-(2,3-dihydroxypropyl)isophthalic acid monoamide (23.1 g, 0.047 moles) **(example 4c)** in ethyl acetate (100 mL), dimethylaminopyridine (567 mg) was added with stirring. The mixture was heated at solvent reflux for ten minutes. Then acetic anhydride (18.2 mL) was added. The heating was continued for four hours. The reaction process was followed by TLC (toluene/ethyl acetate/methanol/dioxane/acetic acid 3:3:1.8:1.4:1). Ethanol (12 mL) was added and the reflux was continued for half hour. The solvent was removed by evaporation to dryness under reduced pressure. The residue was dissolved in methanol (100 mL) and precipitated over ethyl ether (1.5 L). A brown solid was filtered, and dried in vacuo to obtain 5-amino-2,4,6-tribromo-N-(2,3-diacetoxypropyl)isophthalic acid monoamide (24.7 g 91%). This product was purified by PLC (Preparative Liquid Chromatography) (eluent: water).
- ¹H-NMR (200MHz, DMSO-d₆):: δ=2.00 (s, 3H, methyl group), 2.01 (s, 3H, methyl group), 3.35/3.45 (m/m, 2H, CH₂-N), 4.15/4.25 (m/m, 2H, CH₂-O), 5.05 (m, 1H, CH), 5.34 (m, 2H, NH₂), and 8.70 (m, 1H, NH).
- ¹³C-NMR (200MHz, DMSO-d₆):: δ=20.88 (CH₃), 21.27 (CH₃), 38.83 (NH-CH₂), 63.32 (CH₂OH), 70.06 (CH), 100.66/101.23/103.99 (C-Br), 139.69 (aromatic C1), 142.42 (aromatic C3), 147.73 (aromatic C5), 167.15 (CO in CONH), 168.56 (COOH), 170.45/170.74 (CO in COCH₃).

### e) 5-Amino-2,4,6-tribromo-3-[N-(2,3-diacetoxypropyl) carbamoyl]benzoyl chloride

To a solution of 5-amino-2,4,6-tribromo-N-(2,3-diacetoxypropyl)isophthalic acid monoamide (28.7 g, 0.05 moles) **(example 4d)** in ethyl acetate (270 mL), thionyl chloride (15 mL) was added with stirring. The mixture was heated at solvent reflux for six hours. The excess of thionyl chloride was removed by distillation. Then the solvent was evaporated to dryness under reduced pressure. The residue was partitioned in water (290 mL) and chloroform (290 mL). The aqueous phase was extracted with chloroform (1x60mL) and the combined organic extracts were washed with 5% aqueous sodium bicarbonate (1x250mL) and brine (1x250mL). The organic solution was dried over magnesium sulfate overnight. The magnesium sulfate was removed by filtration and the solvent was evaporated to dryness under reduced pressure. The residue was dried in vacuo to obtain 5-amino-2,4,6-tribromo-3-[N-(2,3-diacetoxypropyl)carbamoyl]benzoyl chloride (27:1 g, 92%).
- ¹H-NMR (200MHz, DMSO-d₆):: δ=2.08 (s, 6H, two methyl groups), 3.55/3.75 (m/m, 2H, CH₂N), 4.25/4.35 (m/m, 2H, CH₂-O), 5.05 (bs, 2H, NH₂), 5.23 (m, 1H, CH), and 6.54 (m, 1H, NH).
- ¹³C-NMR (200MHz, DMSO-d₆):: δ=20.66 (CH₃), 20.98 (CH₃), 39.78 (CH₂-N), 62.83 (CH₂-O), 70.03 (CH), 99.80/102.88/107/20 (C-Br), 139, 85 (aromatic C6), 140.73 (aromatic C4), 142.71 (aromatic C2), 165.54 (CO in CONH), 166.25 (CO in COCl), and 170.38/170.74 (CO in COCH₃).

### f) 5-Acetoxyacetylamino-2,4,6-tribromo-3-[N-(2,3-diacetoxypropyl)carbamoyl] benzoyl chloride

To a solution of 5-amino-2,4,6-tribromo-3-[N-(2,3-diacetoxypropyl)carbamoyl]benzoyl chloride (20.6 g, 0.035 moles) (**example 4e**) in dry DMA (125 mL) at 0-5°C, acetoxyacetyl chloride (14.21 g, 0.104 moles) in dry DMA (18 mL) was added dropwise. The reaction medium was stirred at room temperature overnight. The reaction was added over ethyl acetate (208 mL) and water (382 mL). The aqueous layer was extracted with ethyl acetate (2x105 mL), the combined organic extracts were washed with 5% aqueous sodium bicarbonate (2x105 mL), brine (4x105 mL) and dried over magnesium sulfate overnight. After filtration, the solvent was removed by evaporation to dryness under reduced pressure. The residue was treated with chloroform (25 mL). A white solid was filtered and dried in vacuo to obtain 5-acetoxyacetylamino-2,4,6-tribromo-3-[N-(2,3-diacetoxypropyl)carbamoyl]benzoyl chloride (19.5 g, 81%). The product was used in the next step without purification.
- ¹H-NMR (200MHz, DMSO-d₆):: δ=2.01 (s, 6H, two methyl groups), 2.13 (s, 3H, methyl group), 3.60 (m, 2H, CH₂-N), 4.10 (m, 2H, CH₂-O), 4.71 (s, 2H, OCH₂CO), 5.10 (m, 1H, CH), 9.10 (m, 1H, NH), and 10.30 ppm (m,1H, NH).
- ¹³C-NMR (200MHz, DMSO-d₆):: δ= 20.54/20.91 (methyl groups), 39.78 (CH₂-N), 61.87 (CH₂-O), 62.84 (CH), 69.58 (O-CH₂-CO), 113.19/119.29/122.34 (C-Br), 135.85/139.77/141.19 (aromatic C), 164.52 (CO in CON), 165.12 (CO in COCl), 166.12 (CO in CH₂CONH), and 169.72/169.84/170.15 (COCH₃).

### g) 5-Acetoxyacetylamino-2,4,6-tribromo-N-(2,3-dihydroxypropyl)-N-methyl-N'-(2,3-diacetoxypropyl)isophthalamide

To a solution of 5-acetoxyacetylamino-2,4,6-tribromo-3-[N-(2,3-diacetoxypropyl)carbamoyl]benzoyl chloride (19.54 g, 0.028 moles) **(example 4f)** in acetone (265 mL), Na₂CO₃.10H₂O (8.87 g, 0.031 moles) and 3-methylamino-1,2-propanediol (3.26 g, 0.031 moles) were added. The mixture was heated at reflux for four hours. The reaction medium was cooled at room temperature and filtered. The filtrate was evaporated to dryness under reduced pressure to obtain 5-acetoxyacetylamino-2,4,6-tribromo-N,N'-bis-(2,3-diacetoxypropyl)-N-methylisophthalamide (21 g , 98%). This product was used in next step without purification.
- ¹H-NMR (200MHz, DMSO-d₆):: δ = 2.00 (s, 6H, two methyl groups), 2.11 (s, 3H, one methyl group), 3.10-4.30 (m, 9H, four methylene groups and CH), 2.80/3.05 (s/s 3H, CH₃-N), 4.60 (m, 1H, OH), 4.70 (s, 2H, O-CH₂-CO), 4.80 (m, 1H, OH), 5.10 (m, 1H, CH), 9.10, (m, 1H, NH), and 10.20 ppm (m, 1H, NH).
- ¹³C-NMR (200MHz, DMSO-d₆):: δ = 20.67 (CH₃), 20.74 (CH₃), 21.12 (CH₃), 37.17 (CH₃-N), 39.68 (CH₂N), 50.29 (CH₂ in CH₂NCH₃), 62.06 (CH₂ in CH₂OAc), 63.11 (CH₂OH), 64.18 (CH-OAc and CH-OH), 69.93 (CH₂ in O-CH₂CO), 122.01/122.25/122.30 (C-Br), 135.94/140.32/141.44 (aromatic C), 165.55 (CO in CONH), 165.87 (CO in CH₂CONH), 166.12 (CO in CON), and 170.15/170.27/170.58 (CO in acetyl groups).

### h) 2,4,6-Tribromo-5-hydroxyacetylamino-N,N'-bis-(2,3-dihydroxypropyl)-N-methylisophthalamide

To a solution of 5-acetoxyacetylamino-N,N'-bis-(2,3-diacetoxypropyl)-2,4,6-tribromo-N-methyl-isophthalamide (22.6 g, 0.030 moles) **(example 4g)** in methanol/water 1:1 (210 mL) at 50°C, a solution of 20% NaOH was added until stable pH=11. The temperature was kept for an additional hour and cooled at room temperature. The reaction medium was neutralized with hydrochloric acid/water 1:1. The solvents were evaporated to dryness under reduced pressure. The residue was purified by PLC (eluent: 5% methanol) to obtain 2,4,6-tribromo-5-hydroxyacetylamino-N,N'-bis(2,3-dihydroxypropyl)-N-methylisophthalamide (14 g, 74%).
- ¹H-NMR (200MHz, DMSO-d₆):: δ=2.80-3.80 (m, 9H, four methylene groups and CH), 2.86/3.05 (s/s, 3H, CH₃-N), 4.00 (s, 2H, OCH2-CO), 4.60 (m, 2H, two hydroxyl groups), 4.70 (m, 1H, CH), 4.80 (m, 2H, two hydroxyl groups), 5.79 (m, 1H, OH), 8.75 (m, 1H, NH), and 9.83 (m, 1H, NH).
- ¹³C-NMR (200MHz, DMSO-d₆):: δ=33.80 (CH₃), 42.70 (CH₂ in NH-CH₂-), 50.48 (CH₂ in MeN-CH₂-), 61.80 (O-CH₂-CO), 64.10 (CH₂OH), 64.40 (CH₂OH), 70.17 (CH), 70.35 (CH), 115.82/121.86/122.57 (C-Br), 136.84/140.21/141.85 (aromatic C), 165.66 (CO in Ar-CONH), 166.33 (CO in CONH-Ar), and 171.53 (CO in CON).

### Example 5

### Synthesis of 2,4,6-tribromo-5-hydroxyacetylamino-N,N'-bis-(2,3-dihydroxypropyl)isophthalamide

### a) 5-Amino-2,4,6-tribromoisophthalic acid

To a suspension of 5-aminoisophthalic acid (36.2 g, 0.2 mol) in water (1200 mL) was added sufficient concentrated hydrochloric acid to complete solution (approximately pH=1). Bromine (33.8 ml, 0.65 moles) was added dropwise over one hour and the reaction medium stirred at room temperature for two hours. The pH was adjusted to approximately 6 with 50% aqueous sodium hydroxide (100 mL) and then treated with sodium hydrosulfite (6.9 g) to decolour the resulting solution. Sufficient hydrochloric acid was added to adjust pH to 1 and the precipitate was filtered, washed with water, and dried in vacuo to obtain 5-amino-2,4,6-tribromoisophthalic acid (80 g, 96%). The product was used in the next step without purification.
- ¹³C-NMR (200MHz, DMSO-d₆):: δ= 113.90/117.01 (C-Br), 138.56 (C-COOH), 147.34 (C-NH₂), 170.32 (CO in COOH).

### b) 5-Amino-2,4,6-tribromoisophthaloyl dichloride

To a suspension of of 5-amino-2,4,6-tribromoisophthalic acid (41.8 g, 0.1 moles) **(example 5a)** in ethyl acetate (350 mL), thionyl chloride (30 mL) was added with stirring. The mixture was heated at solvent reflux for four hours. Then the reaction medium was cooled at room temperature and the solvent was concentrated until 120 mL. The product, 5-amino-2,4,6-tribromoisophthaloyl dichloride, was filtered, washed with cold ethyl acetate, and dried in vacuo (39 g, 86%). The product was used in the next step without purification.
- ¹³C-NMR (200MHz, DMSO-d₆):: δ= 116.46 (C2-Br), 121.93 (C4/C6-Br), 141.28 (C-COCl), 148.5 (C-NH₂), and 166.73 (CO in COCl).

### c) 5-Acetoxyacetylamino-2,4,6-tribromoisophthaloyl dichloride

A solution of 5-amino-2,4,6-tribromoisophthaloyl dichloride (45.5 g, 0.1 moles) (**example 5b**) in dry DMA (300 mL) was cooled at 0-5°C. To this solution, acetoxyacetyl chloride (41 g, 0.3 moles) in dry DMA (40 mL) was added with stirring in the course of 30 minutes. Then the temperature was allowed to rise to room temperature and the stirring was continued overnight. The reaction was added over ethyl acetate (400 mL) and water (500 mL). The aqueous layer was extracted with ethyl acetate (2x200 mL), the combined organic extracts were washed with 5% sodium bicarbonate (2x105mL), brine (4x105 mL) and dried over magnesium sulfate overnight. After filtration, the solvent was removed by evaporation to dryness under reduced pressure. The crude was treated with ethyl ether (100 mL), filtered, and dried in vacuo to obtain 5-acetoxyacetylamino-2,4,6-tribromoisophthaloyl dichloride (45 g, 81%). The product was used in the next step without further purification.
- ¹H-NMR (200MHz, DMSO-d₆):: δ= 2.12 (s, 3H, CH₃), 4.70 (s, 2H, CH₂), and 10.35 (m, 1H, NH).
- ¹³C-NMR (200MHz, DMSO-d₆):: δ= 20.59 (CH₃), 61.94 (CH₂), 113.53/113.60/121.30 (C-Br), 135.98 (C-NH), 139.98 (C-COCl), 165.97 (COCl), 166.05 (CONH), and 169.84 (COCH₃).

### d) 5-Acetoxyacetylamino-2,4,6-tribromo-N,N'-bis-(2,3-dihydroxypropyl)isophthalamide

To a solution of 5-acetoxyacetylamino-2,4,6-tribromoisophthaloyl dichloride (27.7 g, 0.05 moles) **(example 5c)** in acetone (400 mL), sodium carbonate decahydrate (31.5 g, 0.11 moles) and 3-amino-1,2-propanediol (10.0 g, 0.11 moles) in acetone acetone (10 mL) were added. The mixture was heated at solvent reflux for three hours (the process was followed by TLC: acetone/n-hexane 3:2). The reaction medium was filtered and the solvent was removed by evaporation to dryness under reduced pressure. The crude, 5-acetoxyacetylamino-2,4,6-tribromo-N,N'-bis-(2,3-dihydroxypropyl) isophthalamide, (30 g, 90%), was used in the next step without purification.
- ¹H-NMR (200MHz, DMSO-d₆):: δ= 2.20 (s, 3H, CH₃), 3.29/3.53 (m/m, 4H, 2N-CH₂), 3.38 (m, 4H, 2CH₂-O), 3.85 (m, 2H, 2CH), 4.72 (s, 2H, O-CH₂-CO), 8.90 (m, 2H, 2NH), and 10.10 (m, 1H, NH).
- ¹³C-NMR (200MHz, DMSO-d₆):: δ= 20.56 (CH₃), 41.98/45.05 (N-CH₂), 64.04 (CH₂-O), 66.54 (CH), 70.50 (O-CH₂-CO), 120.40/121.05/12141 (C-Br), 127.41 (C-NH), 136.89/137.12 (C-CONH), 163.40 (CO in CONH), 166.36 (CO in CONH), 168.52 (CO in CH₂-CO), and 169.20 (CO in CH₃-CO).

### e) 2,4,6-Tribromo-5-hydroxyacetylamino-N,N'-bis-(2,3-dihydroxypropyl)isophthalamide

To a solution of 5-acetoxyacetylamino-2,4,6-tribromo-N,N'-bis-(2,3-dihydroxypropyl) isophthalamide (30.0 g, 0.045 moles) **(example 5d)** in methanol/water 1:1 (220 mL) at 50°C, a solution of 20% NaOH was added until stable pH=11. The temperature was kept for an additional hour and cooled at room temperature. The reaction medium was neutralized with hydrochloric acid/water 1:1. The solvents were evaporated to dryness under reduced pressure. The residue was purified by PLC (eluent: 5% methanol) to obtain 2,4,6-tribromo-5-hydroxyacetylamino-N,N'-bis-(2,3-dihydroxypropyl)isophthalamide (21 g, 75%).
- ¹H-NMR (200MHz, DMSO-d₆):: δ= 3.10-3.65 (m, 10H, 4CH₂/2CH), 3.70 (m, 1H, CH), 4.02 (s, 2H; O-CH₂-CO), 4.50 (m, 2H, 2OH), 4.75 (m, 2H, 2OH), 5.80 (m, 1H, OH), 8.60 (m, 2H, 2NH), and 9.85 (m, 1H, NH).
- ¹³C-NMR (200MHz, DMSO-d₆):: δ= 42.55/42.66 (2CH₂-NH), 61.61 (O-CH₂-CO), 63.96/64.07 (2CH₂OH), 70.23/70.36 (2CH), 116.26 (C2-Br), 122.46 (C4/C6-Br), 136.29 (C-NH), 141.24 (2C-CO), 165.62 (CO in CONH), 171.37 (CO in CH₂-CO).

The compound **5e** can be obtained from 5-nitroisophthalic acid dimethyl ester (examples **5f-5j**).

### f) 5-Nitro-N,N'-bis-(2,3-dihydroxypropyl)isophthalamide

To a solution of 5-nitroisophthalic acid dimethyl ester (23.92 g, 0.1 moles) in methanol (100 mL), 3-amino-1,2-propanediol (16.52 g, 0.22 moles) was added with stirring and heated at solvent reflux for four hours. The solution was evaporated to dryness under reduced pressure. The crude was crystallized in methanol/ethyl ether, filtered off, and dried in vacuo to obtain 5-nitro-N,N'-bis-(2,3-dihydroxypropyl)isophthalamide (30.3 g, 85%).
- ¹H-NMR (200MHz, DMSO-d₆):: δ= 3.07-3.55 (m, 5H, CH₂-N, CH₂-O, CH), 4.55 (m, 2H, 2OH), 7.00 (m, 2H, 2NH), 8.05 (s, 1H, aromatic CH), and 8.71 (s, 2H, two aromatic CH).
- ¹³C-NMR (200MHz, DMSO-d₆):: δ= 43.51 (CH₂-N), 66.85 (CH₂-O), 71.09 (CH), 115.27 (two aromatic CH), 131.93 (aromatic CH), 135.30 (C-CONH), 149.02 (C-NO₂), and 163.21 (CO).

### g) 5-Amino-N,N'-bis-(2,3-dihydroxypropyl)isophthalamide

A suspension of 5-nitro-N,N'-bis-(2,3-dihydroxypropyl)isophthalamide (35.7 g, 0.1 moles) **(example 5f)** in water (250 mL) was hydrogenated with 1.8 g of palladium over charcoal at 70°C for three hours. The reaction medium was cooled to room temperature and filtered. The solution was evaporated to dryness under reduced pressure. The residue was treated with methanol (60 mL), filtered, and dried at 50°C in vacuo to obtain 5-amino-N,N'-bis-(2,3-dihydroxypropyl)isophthalamide (30.7 g, 94%).
- ¹H-NMR (200MHz, DMSO-d₆):: δ= 2.70-3.55 (m, 10H, 2CH₂-N, 2CH₂-O, and 2CH), 4.57/4.68 (m/m, 4H, 4OH), 6.95 (m, 2H, 2NH), 7.25 (s, 2H, 2 aromatic CH), 7.43 (s, 1H, aromatic CH).
- ¹³C-NMR (200MHz, DMSO-d₆):: δ= 43.24 (NH-CH₂), 66.18 (CH₂-OH), 69.99 (CH), 120.49 (aromatic CH), 121.36 (aromatic CH), 133.46 (C-CONH), 148.25 (C-NH₂), 165.96 (CO in CONH).

### h) 5-Amino-2,4,6-tribromo-N,N'-bis-(2,3-dihydroxypropyl)-isophthalamide

To a suspension of 5-amino-N,N'-bis-(2,3-dihydroxypropyl)isophthalamide (16.37 g, 0.05 moles) **(example 5g)** in water (150 mL), concentrated hydrochloric acid was added until total dissolution (pH=1.5). This mixture was brominated by adding dropwise bromine (8.5 mL, 26.4 g, 0.165 moles) with stirring at room temperature in the course of 20 minutes. The solution was stirred an additional hour and treated with sodium hydrosulfite until colorless reaction medium. It was evaporated to dryness under reduced pressure. The residue was treated with methanol (60 mL), filtered, and the filtrate evaporated to dryness under reduced pressure, and dried in vacuo to obtain 5-amino-2,4,6-tribromo-N,N'-bis-(2,3-dihydroxypropyl)isophthalamide (23 g, 82%).
- ¹H-NMR (200MHz, DMSO-d₆):: δ= 3.29/3.40/3.53 (m/m/m, 8H, 2CH₂-N and 2CH₂-O), 3.95 (m, 2H, 2CH), 4.65 (m, 4H, 4OH), 5.70 (bs, 2H, NH₂), and 7.95 (m, 2H, 2NH).
- ¹³C-NMR (200MHz, DMSO-d₆):: δ= 43.29 (CH₂-N), 66.29 (CH₂-O), 70.93 (CH), 115.98/120.75 (C-Br), 136.23/136.83/138.27 (aromatic C), and 165.93/166.01 (CO in CONH).

### i) 5-Amino-2,4,6-tribromo-N,N'-bis-(2,3-diacetoxypropyl)-isophthalamide

To a solution of 5-amino-2,4,6-tribromo-N,N'-bis-(2,3-dihydroxypropyl)isophthalamide (28.2 g, 0.05 moles) **(example 5h)** in ethyl acetate (150 mL), dimethylaminopyridine (603 mg) was added with stirring. The mixture was heated at solvent reflux for ten minutes. Then acetic anhydride (19.5 mL) was added. The heating was continued for four hours. The reaction process was followed by TLC (toluene/ethyl acetate/methanol/dioxane/acetic acid 3:3:1.8:1.4:1). Ethanol (13 mL) was added and the reflux was continued for half hour. The solvent was removed by evaporation to dryness under reduced pressure. The residue was purified by precipitation in methanol/ethyl ether (100 mL/1.5 L). A brown solid was filtered, and dried in vacuo to obtain 5-amino-2,4,6-tribromo-N,N'-bis-(2,3-diacetoxypropyl)isophthalamide (31.1 g, 85%).
- ¹H-NMR (200MHz, DMSO-d₆):: δ=2.06 (s, 6H, two methyl groups), 2.13 (s, 6H, two methyl groups), 3.43/3.68 (m/m, 4H, 2CH₂-N), 4.27/4.45 (m/m, 2H, 2CH₂-O), 5.05 (m, 2H, 2CH), 5.29 (m, 2H, NH₂), and 8.58 (m, 2H, 2NH).
- ¹³C-NMR (200MHz, DMSO-d₆):: δ=20.50 (CH₃), 24.10 (CH₃), 40.95 (NH-CH₂), 62.38 (CH₂OH), 69.26 (CH), 113.96/117.23/117.99 (C-Br), 136.69/136.92/138.73 (aromatic C), 162.0/162.715 (CO in CONH), 171.41/171.93 (CO in COCH₃).

### j) 5-Acetoxyacetylmino-2,4,6-tribromo-N,N'-bis-(2,3-diacetoxypropyl)isophthalamide

To a solution of 5-amino-2,4,6-tribromo-N,N'-bis-(2,3-diacetoxypropyl)-isophthalamide (25.6 g, 0.035 moles) **(example 5i)** in dry DMA (125 mL) at 0-5°C, acetoxyacetyl chloride (14.21 g, 0.104 moles) in dry DMA (18 mL) was added dropwise. The reaction medium was stirred at room temperature overnight. The reaction was added over ethyl acetate (208 mL) and water (382 mL). The aqueous layer was extracted with ethyl acetate (2x105 mL), the combined organic extracts were washed with brine (4x105 mL) and dried over magnesium sulfate overnight. After filtration, the solvent was removed by evaporation to dryness under reduced pressure. The residue was treated with chloroform (25 mL). A white solid was filtered and dried in vacuo to obtain 5-acetoxyacetylmino-2,4,6-tribromo-N,N'-bis-(2,3-diacetoxypropyl)isophthalamide (23.3 g, 80%). The product was used in the next step without purification.
- ¹H-NMR (200MHz, DMSO-d₆):: δ=2.06 (s, 3H, methyl group), 2.13 (s, 3H, methyl group), 2.18 (s, 3H, methyl group), 3.50-3.60 (m, 4H, 2CH₂-N), 4.00-4.10 (m, 4H, 2CH₂-O), 4.71 (s, 2H, OCH₂CO), 5.09 (m, 1H, CH), 8.90 (m, 1H, NH), and 9.95 (m,1H, NH).
- ¹³C-NMR (200MHz, DMSO-d₆):: δ= 20.50 (CH₃), 20.61 (CH₃), 24.10 (CH₃), 39.28 (CH₂-N), 62.38 (C-CH₂-O), 63.05 (CH), 70.08 (O-CH₂-CO), 117.42/118.03/120.14 (C-Br), 130.25/135.12/135.50 (aromatic C), 165.12/165.81 (CO in CONH), 167.18 (CO in CONHAr), and 169.20/170.35/172.00 (CO in COCH₃).

The hydrolysis of compound **5j** yields a compound with identical spectroscopic characteristics than the compound **5e.**

### Example 6

### Synthesis of 2,4,6-tribromo-5-hydroxyacetylamino-N-(2,3-dihydroxypropyl)-N'-(2-hydroxyethyl)isophthalamide

### a) 5-Acetoxyacetylamino-2,4,6-tribromo-N-(2,3-diacetoxypropyl)-N-(2-hydroxyethyl)isophthalamide

To a solution of 5-acetoxyacetylamino-2,4,6-tribromo-3-[N-(2,3-diacetoxypropyl)carbamoyl]benzoyl chloride (27.7 g, 0.04 moles) **(example 4f)** in acetone (380 mL), Na₂CO₃.10H₂O (12.6 g, 0.044 moles) and 2-aminoethanol (2.7 g, 0.044 moles) were added. The mixture was heated at reflux for four hours. The reaction medium was cooled at room temperature and filtered off. The filtrate was evaporated to dryness under reduced pressure to obtain 5-acetoxyacetylamino-2,4,6-tribromo-N-(2,3-diacetoxypropyl)-N-(2-hydroxyethyl)isophthalamide (25.8 g, 90%). This compound was used in next step without further purification.
- ¹H-NMR (200MHz, DMSO-d₆):: δ = 2.06 (s, 3H, CH₃), 2.13 (s, 3H, CH₃), 2.18 (s, 3H, CH₃), 3.20-4.30 (m, 8H, 4CH₂), 4.60 (m, 1H, OH), 4.70 (s, 2H, O-CH₂-CO), 5.10 (m, 1H, CH), 9.10 (m, 1H, NH), and 10.20 (m, 1H, NH).
- ¹³C-NMR (200MHz, DMSO-d₆):: δ= 20.50 (CH₃), 20.56 (CH₃), 24.10 (CH₃), 39.25 (CH₂ in N-CH₂-CH₂), 42.58 (CH₂ in N-CH₂-CH), 61.68 (CH₂OH), 62.50 (CH₂OH), 68.43 (CH), 70.01 (O-CH₂-CO), 120.90/121.20/122.17 (C-Br), 127.94 (C-NH), 135.77 (aromatic C3), 137.27 (aromatic C1), 162.05 (CO in CONH), 166.59 (CO in CONH), 168.62 (CO in CH₂CONH), 169.30 (CO in CH₃COCH₂), 170.42 (CO in acetyl group), and 172.00 (CO in acetyl group).

### b) 2,4,6-Tribromo-5-hydroxyacetylamino-N-(2,3-dihydroxypropyl)-N'-(2-hydroxyethyl)isophthalamide

To a solution of 5-acetoxyacetylamino-2,4,6-tribromo-N-(2,3-diacetoxypropyl)-N-(2-hydroxyethyl)isophthalamide (21.5 g, 0.030 moles) **(example 6a)** in methanol/water 1:1 (210 mL) at 50°C, a solution of 20% NaOH was added until stable pH=11. The temperature was kept for an additional hour and cooled at room temperature. The reaction medium was neutralized with hydrochloric acid/water 1:1. The solvents were evaporated to dryness under reduced pressure. The residue was purified by PLC (eluent: 5% methanol) to obtain 2,4,6-tribromo-5-hydroxyacetylamino-N-(2,3-dihydroxypropyl)-N'-(2-hydroxyethyl)isophthalamide (13 g, 73%).
- ¹H-NMR (200MHz, DMSO-d₆):: δ= 3.10-3.65 (m, 9H, 2CH₂-N/CH/2CH₂-O), 4.01 (s, 2H, O-CH₂-CO), 4.55 (m, 1H, OH), 4.70 (m, 2H, 2OH), 5.65 (m, 1H, OH), 8.70 (m, 2H, 2NH), and 9.85 (m, 1H, NH).
- ¹³C-NMR (200MHz, DMSO-d₆):: δ= 41.90 (CH₂ in N-CH₂-CH₂), 42.60 (N-CH₂-CH), 59.54 (CH₂OH), 61.66 (CH₂OH), 64.00 (CH₂OH), 70.29 (CH), 116.27/122.48/122.52 (C-Br), 136.33 (C-NH), 141.26 (aromatic C3), 141.71 (aromatic C1), 165.57 (CO in CONH), 165.71 (CO in CONH), and 171.48 (CO in HO-CH₂-CO).

### Example 7

### Synthesis of 2,4,6-tribromo-5-[N-methyl-hydroxyacetylamino]-N,N'-bis-(2,3-dihydroxypropyl)isophthalamide

### a) 2,4,6-tribromo-5-[N-methyl-acetoxyacetylamino]-N,N'-bis-(2,3-diacetoxypropyl)isophthalamide

To a solution of 5-acetoxyacetylamino-2,4,6-tribromo-N,N'-bis-(2,3-diacetoxypropyl)isophthalamide (25 g, 0.03 moles) **(example 5j)** in acetone (300 mL), sodium carbonate (6.4 g, 0.06 moles), and dimethyl sulfate (7.6 g, 0.06 moles) were added with stirring. The mixture was heated at 50°C for three hours. The sodium carbonate was filtered off and the solvent was removed by evaporation to dryness under reduced pressure. The crude residue was partitioned between water (200 mL) and dichloromethane (200 mL). The aqueous phase was extracted with dichloromethane (2x50 mL) and the combined organic extracts were washed with 5% sodium carbonate (1x100 mL) and brine (1x100 mL). The organic solution was dried over magnesium sulfate overnight. After filtration, the solvent was removed by evaporation to dryness under reduced pressure to obtain 2,4,6-tribromo-5-[N-methyl-acetoxyacetylamino]-N,N'-bis-(2,3-diacetoxypropyl)isophthalamide (20.1 g, 79%). The product was used in the next step without further purification.
- ¹H-NMR (200MHz, DMSO-d₆):: δ= 2.06 (s, 6H, 2CH₃), 2.10 (s, 6H, 2CH₃), 2.20 (s, 3H, CH₃), 3.00/3.20 (s/s, 3H, CH₃-N), 3.40/3.65 (m/m, 4H, 2CH₂-N), 4.35/4.55 (m/m, 4H, 2CH₂-O), 4.65 (s, 2H, O-CH₂-CO), 5.10 (m, 2H, 2CH), and 8.32 (m, 2H, 2NH).
- ¹³C-NMR (200MHz, DMSO-d₆):: δ = 20.56 (CH₃), 20.65 (CH₃), 24.10 (CH₃), 40.00 (CH₂-N), 42.23 (CH₃-N), 42.68 (CH₂-N), 62.39 (CH₂-O), 68.51 (CH), 73.19 (O-CH₂-CO), 123.74/130.15 (C-Br), 140.22 (aromatic C3), 140.81 (aromatic C1), 146.14 (C-N), 162.05 (CO in CONH), 163.21 (CO in CONH), 165.93 (CO in CON), 169.20 (CO in COCH₃), 170.35 (CO in COCH₃), and 172.10 (CO in COCH₃).

### b) 2,4,6-tribromo-5-[N-methyl-hydroxyacetylamino]-N,N'-bis-(2,3-dihydroxypropyl)isophthalamide

To a solution of 2,4,6-tribromo-5-[N-methyl-acetoxyacetylamino]- N,N'-bis-(2,3-diacetoxy-propyl)isophthalamide (16.9 g, 0.02 moles ) **(example 7a)** in methanol/water 1:1 (200 mL) at 50°C, a solution of 20% NaOH was added until stable pH=11. The temperature was kept for an additional hour and cooled at room temperature. The reaction medium was neutralized with hydrochloric acid/water 1:1. The solvents were evaporated to dryness under reduced pressure. The residue was purified by PLC (eluent: 5% methanol) to obtain 2,4,6-tribromo-5-[N-methyl-hydroxyacetylamino]-N,N'-bis-(2,3-dihydroxypropyl) isophthalamide (10 g, 79%).
- ¹H-NMR (200MHz, DMSO-d₆):: δ = 3.00/3.34 (s/s, 3H, CH₃-N), 3.20/3.52 (m/m, 4H, 2CH₂-N), 3.40 (m, 4H, 2CH₂-O), 4.09 (s, 2H, O-CH₂-CO), 4.89 (m, 2H, 2CH), 4.90-5-10 (m, 5H, 5OH), and 8.40 (m, 2H, 2NH).
- ¹³C-NMR (200MHz, DMSO-d₆):: δ = 40.91 (CH₃), 41.98 (CH₂-N), 44.82 (CH₂-N), 62.00 (O-CH₂-CO), 66.32 (CH₂-O), 71.23 (CH), 122.21/129.22/129.81 (C-Br), 139.51 (aromatic C1/C3), 144.97 (C-N), 163.40 (CO in CONH), 166.36 (CO in CONH), and 169.93 (CO in CH₃CO).

### Example 8

### Synthesis of 2,4,6-tribromo-5-[N-(2-hydroxyethyl)hydroxyacetylamino]-N,N'-bis-(2,3-dihydroxypropyl) isophthalamide

### a) 2,4,6-Tribromo-5-[N-(2-hydroxyethyl)acetoxyacetylamino]-N,N'-bis-(2,3-diacetoxypropyl)isophthalamide

To a solution of 5-acetoxyacetylamino-2,4,6-tribromo-N,N'-bis-(2,3-diacetoxypropyl)isophthalamide (25 g, 0.03 moles) **(example 5j)** in acetone (300 mL), sodium carbonate (5.3 g, 0.05 moles), and 2-chloroethanol (4.02 g, 0.05 moles) were added with stirring. The mixture was heated at 50°C for five hours. The sodium carbonate was filtered off and the solvent was removed by evaporation to dryness under reduced pressure. The crude residue was partitioned between water (250 mL) and dichloromethane (250 mL). The aqueous phase was extracted with dichloromethane (2x60 mL) and the combined organic extracts were washed with 5% aqueous sodium carbonate (1x150 mL) and brine (1x150 mL). The organic solution was dried over magnesium sulfate overnight. After filtration, the solvent was removed by evaporation to dryness under reduced pressure to obtain 2,4,6-tribromo-5-[N-(2-hydroxyethyl)acetoxyacetylamino]-N,N'-bis-(2,3-diacetoxypropyl)isophthalamide (20.1 g, 79%). The product was used in the next step without further purification.
- ¹H-NMR (200MHz, DMSO-d₆):: δ= 2.10 (s, 6H, 2CH₃), 2.17 (s, 6H, 2CH₃), 2.21 (s, 3H, CH₃), 3.43/3.69 (m/m, 8H, CH₂OH, 2CH₂NH, CH₂N), 4.60 (m, 4H, CH₂OCO), 4.70 (s, 2H, O-CH₂-CO), 5.03 (m, 2H, 2CH), 8.19 (m, 2H, 2NH).
- ¹³C-NMR (200MHz, DMSO-d₆):: δ= 20.23 (CH₃), 20.53 (CH₃), 24.01 (CH₃), 39.68 (CH₂-NH), 40.27 (CH₂-NH), 47.80 (CH₂-N), 58.30 (CH₂OH), 62.24 (CH₂-OCO), 68.37 (CH), 71.29 (O-CH₂-CO), 123.96/129.18 (C-Br), 140.21 (C-CONH), 145.53 (C-CON), 164.33/164.86 (CO), 169.10/169.35/171.19 (CO in COCH₃).

### b) 2,4,6-Tribromo-5-[N-(2-hydroxyethyl)hydroxyacetylamino]-N,N'-bis-(2,3-dihydroxypropyl)isophthalamide

To a solution of 2,4,6-tribromo-5-[N-(2-hydroxyethyl)acetoxyacetylamino]-N,N'-bis-(2,3-diacetoxypropyl)isophthalamide **(example 8a)** (17.5 g, 0.02 moles ) in methanol/water 1:1 (200 mL) at 50°C, a solution of 20% NaOH was added until stable pH=11. The temperature was kept for an additional hour and cooled at room temperature. The reaction medium was neutralized with hydrochloric acid/water 1:1. The solvents were evaporated to dryness under reduced pressure. The residue was purified by PLC (eluent: 5% methanol) to obtain 5-[N-(hydroxyacetyl)-N-(2-hydroxyethyl)amino]-2,4,6-tribromo-N,N'-bis-(2,3-dihydroxy-propyl)isophthalamide (11.3 g, 85%).
- ¹H-NMR (200MHz, DMSO-d₆):: δ= 3.20-3.75 (m, 12 H, 3CH₂-N/3CH₂-O), 4.09 (s, 2H, O-CH₂-CO), 4.65/4.80 (m/m, 4H, 40H), 4.60 (m, 2H, 2CH), 5.53 (m, 2H, 2OH), 8.63 (m, 2H, 2NH).
- ¹³C-NMR (200MHz, DMSO-d₆):: δ= 42.03 (CH₂-NH), 42.51 (CH₂-NH), 45.23 (CH₂-N), 58.30 (CH₂-OH), 61.21 (O-CH₂-CO), 66.54 (CH₂-OH), 71.04 (CH), 122.45/128.07/128.32 (C-Br), 139.02/139.43 (C-CONH), 145.17 (C-N), 164.81/165.03 (CO in CONH), 172.49 (CO in CON).

### Example 9

### Synthesis of 2,4,6-tribromo-5-[N-(2,3-dihydroxypropyl) hydroxyacetylamino]-N,N'-bis-(2,3-dihydroxypropyl)isophthalamide

### a) 2,4,6-Tribromo-5-[N-(2,3-dihydroxypropyl)acetoxyacetylamino]-N,N'-bis-(2,3-diacetoxypropyl)isophthalamide

To a solution of 5-acetoxyacetylamino-2,4,6-tribromo-N,N'-bis-(2,3-diacetoxypropyl)isophthalamide (25 g, 0.03 moles) **(example 5j)** in acetone (300 mL), sodium carbonate (6.36 g, 0.06 moles), and 2-chloroethanol (6.63 g, 0.06 moles) were added with stirring. The mixture was heated at 50°C for five hours. The sodium carbonate was filtered off and the solvent was removed by evaporation to dryness under reduced pressure. The crude residue was partitioned between water (250 mL) and dichloromethane (250 mL). The aqueous phase was extracted with dichloromethane (2x60 mL) and the combined organic extracts were washed with 5% sodium carbonate (1x150 mL) and brine (1x150 mL). The organic solution was dried over magnesium sulfate overnight. After filtration, the solvent was removed by evaporation to dryness under reduced pressure to obtain 2,4,6-tribromo-5-[N-(2,3-dihydroxypropyl)acetoxyacetylamino]-N,N'-bis-(2,3-diacetoxypropyl)isophthalamide (22.3 g, 82%). The product was used in the next step without posterior purification.
- ¹H-NMR (200MHz, DMSO-d₆):: δ= 2.06 (s, 6H, 2CH₃), 2.13 (s, 6H, 2CH₃), 2.18 (s, 3H, CH₃), 3.44/3.68/3.82 (m/m/m, 8H, CH₂OH, 2CH₂NH, CH₂N), 4.50 (m, 4H, CH₂OCO), 4.71 (s, 2H, O-CH₂-CO), 5.10 (m, 2H, 2CH), 7.79 (m, 2H, 2NH).
- ¹³C-NMR (200MHz,DMSO-d₆):: δ= 20.50 (CH₃), 20.64 (CH₃), 23.01 (CH₃), 40.21 (CH₂-NH), 41.93 (CH₂-NH), 47.80 21 (CH₂-N), 61.24 (CH₂-OCO), 66.03 (CH₂-OH), 68.21 (CH),70.82 (CH), 74.09 (O-CH₂-CO), 123.81/129.91 (C-Br), 140.21/140.53 (C-CONH), 145.09 (C-CON), 162.33/164.86/165.03 (CO), 169.20/170.35/172.00 (CO in COCH₃).

### b) 2,4,6-Tribromo-5-[N-(2,3-dihydroxypropyl)hydroxyacetylamino]-N,N'-bis-(2,3-dihydroxypropyl)isophthalamide

To a solution of 2,4,6-tribromo-5-[N-(2,3-dihydroxypropyl)-acetoxyacetylamino]-N,N'-bis-(2,3-diacetoxypropyl)-isophthalamide **(example 9a)** (18.13 g, 0.02 moles ) in methanol/water 1:1 (200 mL) at 50°C, a solution of 20% NaOH was added until stable pH=11. The temperature was kept for an additional hour and cooled at room temperature. The reaction medium was neutralized with hydrochloric acid/water 1:1. The solvents were evaporated to dryness under reduced pressure. The residue was purified by PLC (eluent: 5% methanol) to obtain 2,4,6-tribromo-5-[N-(2,3-dihydroxypropyl)-hydroxyacetylamino]-N,N'-bis-(2,3-dihydroxypropyl)isophthalamide (11.8 g, 85%).
- ¹H-NMR (200MHz, DMSO-d₆):: δ= 3.20-3.60 (m, 12 H, 3CH₂-N/3CH₂-O), 4.11 (s, 2H, O-CH₂-CO), 4.65/4.80 (m/m, 4H, 4OH), 4.70/4.75 (m/m, 3H, 3CH), 5.53 (m, 3H 3OH), 8.82 (m, 2H, 2NH).
- ¹³C-NMR (200MHz, DMSO-d₆):: δ= 41.98/42.09 (CH₂-NH), 45.37 (CH₂-N), 61.85 (O-CH₂-CO), 66.54/66.88 (CH₂-OH), 70.84/71.04 (CH), 122.22/129.20/129.78 (C-Br), 139.29/140.05 (C-CONH), 144.74 (C-N), 163.40/163.99 (CO in CONH), 172.26 (CO in CON).

### Example 10

### Synthesis of 2,4,6-tribromo-5-bromomethanesulfonylamino-N,N'-bis-(2,3-dihydroxypropyl)-N-methylisophthalamide

### a) 2,4,6-tribromo-5-bromomethanesulfonylamino-3-[N-(2,3-diacetoxypropyl)carbamoyl]benzoyl chloride

To a solution of 5-amino-2,4,6-tribromo-3-[N-(2,3-diacetoxypropyl)carbamoyl]benzoyl chloride (17.8 g, 0.03 moles) **(example 4e)** in dry DMA (100 mL) at 0-5°C, bromomethanesulfonyl bromide (21.4 g, 0.09 moles) in dry DMA (15 mL) was added dropwise. The reaction medium was stirred at room temperature overnight. The reaction was added over ethyl acetate (200 mL) and water (350 mL). The aqueous layer was-extracted with ethyl acetate (2x90 mL), the combined organic extracts were washed with brine (4x90 mL) and dried over magnesium sulfate overnight. After filtration, the solvent was removed by evaporation to dryness under reduced pressure. The crude was purified by flash chromatography (ethyl acetate) to obtain 2,4,6-tribromo-5-bromomethanesulfonylamino-3-[N-(2,3-diacetoxypropyl)carbamoyl]benzoyl chloride (15.7 g, 70%).
- ¹H-NMR (200MHz, DMSO-d₆):: δ= 2.16 (s, 3H, CH₃), 2.21 (s, 3H, CH₃), 3.43/3.70 (m/m, 2H, CH₂-N), 4.30/4.50 (m/m, 2H, CH₂-O), 4.75 (s, 2H, CH₂-SO₂), 5.40 (m, 1H, CH), 7.20 (m, 1H, NH), and 7.43 (m, 1H, NH in SO₂NH).
- ¹³C-NMR (200MHz, DMSO-d₆):: δ= 20.50 (CH₃), 24.10 (CH₃), 39.68 (CH₂-N), 48.52 (CH₂-SO₂), 62.63 (CH₂-O), 69.04 (CH), 120.01/123.19/123.47 (C-Br), 133.56 (C-NH), 138.93 (C-COCl), 143.67 (C-CONH), 165.22 (CO in CONH), 166.73 (CO in COCI), 170.35 (CO in COCH₃), and 172.00 (CO in COCH₃).

### b) 2,4,6-Tribromo-5-bromomethanesulfonylamino-N-(2,3-dihydroxypropyl)-N-methyl-N'-(2,3-diacetoxypropyl) isophthalamide

To a solution of 2,4,6-tribromo-5-bromomethanesulfonylamino-3-[N-(2,3-diacetoxypropyl)carbamoyl]benzoyl chloride (22.51 g, 0.03 moles) (**example 10a)** in acetone (250 mL), Na₂CO₃.10H₂O (9.45 g, 0.033 moles) and 3-methylamino-1,2-propanediol (3.47 g, 0.033 moles) were added. The mixture was heated at reflux for four hours. The reaction medium was cooled at room temperature and filtered. The filtrate was evaporated to dryness under reduced pressure and dried in vacuo to obtain 2,4,6-tribromo-5-bromomethanesulfonylamino-N-(2,3-dihydroxypropyl)-N-methyl-N'-(2,3-diacetoxypropyl)-isophthalamide (23.3 g, 95%). This compound was used in next step without purification.
- ¹H-NMR (200MHz, DMSO-d₆):: δ= 2.06 (CH₃), 2.17 (CH₃), 2.83/3.07 (s/s, 3H, CH₃-N), 3.40/3.70/3.85 (m/m/m, 6H, 2CH₂-N and CH₂-O), 4.40/4.55 (m/m, 2H, CH₂-OAc), 4.77 (s, 2H, CH₂-SO₂), 5.30-5.40 (m, 2H, 2CH), 6.70 (m, 1H, NH), 7.78 (m, 1H, NH), and 7.52 (m, 1H, NH-SO₂).
- ¹³C-NMR (200MHz, DMSO-d₆):: δ= 20.81 (CH₃), 23.97 (CH₃), 33.99/37.10 (CH₃-N), 39.68 (CH₂-N), 51.53 (CH₂-NMe), 62.38 (CH₂-O), 67.05 (CH₂-O), 68.37 (CH), 69.39 (CH), 118.33/121.51/122.31 (C-Br), 128.44 (C-NH), 138.54 (C-CONH), 138.62 (C-CON), 165.00 (CO in CONH), 169.53 (CO in CON), 170.35 (CO in COCH₃), and 172.01 (CO in COCH₃).

### c) 2,4,6-Tribromo-5-bromomethanesulfonylamino-N,N'-bis-(2.3-dihydroxypropyl)-N-methylisophthalamide

To a solution of 2,4,6-tribromo-5-bromomethanesulfonylamino-N-(2,3-dihydroxypropyl)-N-methyl-N'-(2,3-diacetoxypropyl)isophthalamide (20.48 g, 0.025 moles) **(example 10b)** in methanol/water 1:1 (200 mL) at 50°C, a solution of 20% NaOH was added until stable pH=11. The temperature was kept for an additional hour and cooled at room temperature. The reaction medium was neutralised with hydrochloric acid/water 1:1. The solvents were evaporated to dryness under reduced pressure. The residue was purified by PLC (eluent: 5% methanol) to obtain 2,4,6-tribromo-5-bromomethanesulfonylamino-N,N'-bis(2,3-dihydroxypropyl)-N-methylisophthalamide (14 g, 74%).
- ¹H-NMR (200MHz, DMSO-d₆):: δ= 2.90/3.10 (s/s, 3H, CH₃-N), 3.25/3.53/ 3.40/3.80 (m/m/m/m, 8H, 2CH₂-N and 2CH₂-O), 4.80 (s, 2H, CH₂-SO₂), 4.90/4.97 (m/m, 2H, 2CH), 7.85 (m, 1H, NH), and 7.80 (m, 1H, NH-SO₂).
- ¹³C-NMR (200MHz, DMSO-d₆):: δ= 34.22/36.97 (CH₃-N), 41.98 (CH₂-N), 48.73 (CH₂-SO₂), 50.83/53.50 (CH₂-NMe), 64.28 (CH₂-O), 64.83 (CH₂-O), 69.93 (CH), 70.21 (CH), 117.13/121.74/122.08 (C-Br), 132.24 (C-NH), 139.83 (aromatic C3), 140.22 aromatic C1), 165.84 (CO in CON), and 170.20 (CO in CONH).

### Example 11

### Synthesis of 2,4,6-tribromo-5-hydroxyacetylamino-N-(2,3-dihydroxypropyl)-N-methylisophthalamide

### a) 5-Nitroisophthalamic acid

5-Nitroisophthalic acid monomethyl ester (20 g, 0.09 moles) was added portionwise to a mechanical stirred ammonium hydroxide solution (100 mL), in five minutes keeping the temperature below 25°C. After 15-30 minutes the clear solution began to precipitate and 1-1.5 hours later a very thick mixture which blocks the stirrer was formed. This mixture was allowed to stand for additional two hours to complete the reaction. The thick mixture was diluted with water (60 mL). Then concentrated hydrochloric acid was added to keep the pH<1. The temperature raised spontaneously to 85-90°C and this temperature was maintained by heating (almost all the solid was dissolved). The reaction was cooled at 65°C and the pH adjusted to 2 with 25% ammonium hydroxide solution. When the temperature was below 25°C, the crystallised solid was filtered off, washed with water until negative chloride test, and dried overnight at 70°C. to obtain 5-nitroisophthalamic acid (17 g, 90%). This compound was used in next step without further purification.
- ¹H-NMR (200MHz, DMSO-d₆):: δ= 6.25 (bs, 2H, NH₂), 7.72/8.63/8.86 (aromatic CH).
- ¹³C-NMR (200MHz, DMSO-d₆):: δ= 110.27/129.36/136.41 (CH), 134.10 (C-COOH), 135.51 (C-CONH₂), 149.83 (C-NO₂), 164.97 (CO in CONH₂), and 165.96 (CO in COOH).

### b) 5-Aminoisophthalamic acid

To a suspension of 5-nitroisophthalamic acid (18.9 g, 0.09 moles) **(example 11a)** in water (90 mL) at 50°C., 25% aqueous ammonium hydroxide (5.5 mL) was added dropwise (the pH increased from 1.53 to 3.35). The mixture was diluted with water (90 mL) and hydrogenated with 1.5 g of palladium over charcoal at 70°C for three hours. The reaction medium was cooled to room temperature and filtered off. Hydrochloric acid was added (pH=2.5) and the solution was evaporated to dryness under reduced pressure. The residue was treated with methanol (50 mL), filtered, and dried at 50°C in vacuo to obtain 5-aminoisophthalamic acid (14.8 g, 91%). This compound was used in next step without further purification.
- ¹H-NMR (200MHz, DMSO-d₆):: δ= 4.90(m, 2H, NH2), 6.10 (bs, 2H, NH₂ in CONH₂), 7.18/7.82/8.09 (s/s/s, aromatic CH).
- ¹³C-NMR (200MHz, DMSO-d₆):: δ=119.33/120.81/125.93 (aromatic CH), 130.28 (C-COOH), 134.14 (C-CONH₂), 147.03 (C-NH₂),), 166.70 (CO in CONH₂), and 167.79 (CO in COOH).

### c) 5-Amino-2,4,6-tribromoisophthalamic acid

To a solution of 5-aminoisophthalamic acid (18 g, 0.1 moles) **(example 11b)** in water (900 mL), bromine (16.7 mL, 0.325 moles) was added dropwise with stirring at room temperature in the course of half hour.

The solution was stirred an additional hour and adjusted to pH=9 with sodium hydroxide. The pH was decreased to 4.2 with sodium hydrosulphite. This operation was repeated until clear reaction medium. It was evaporated to dryness under reduced pressure. The residue was treated with methanol (90 mL) and filtered. The filtrate was evaporated to dryness under reduced pressure. The residue was treated with isopropanol (90 mL) and filtered. The solid was dried in vacuo at room temperature to obtain. 5-amino-2,4,6-tribromoisophthalamic acid (37.9 g, 91%). This compound was used in next step without further purification.
- ¹³C-NMR (200MHz, DMSO-d₆):: δ = 115.68/117/05/117.98 (C-Br), 134.82 (C-COOH), 140.24 (C-CONH₂), 142.13 (C-NH₂), 167.58 (CO in CONH₂), and 168.93 (CO in COOH).

### d) 5-Amino-2,4,6-tribromo-3-carbamoylbenzoyl chloride

To a suspension of 5-amino-2,4,6-tribromoisophthalamic acid (20.84 g, 0.05 moles) **(example 11c)** in ethyl acetate (230 mL), thionyl chloride (15 mL) was added with stirring. The mixture was heated at solvent reflux for six hours. The excess of thionyl chloride was removed by distillation. Then the solvent was evaporated to dryness under reduced pressure. The residue was partitioned between water (250 mL) and chloroform (250 mL). The aqueous phase was extracted with chloroform (1x50mL) and the combined organic extracts were washed with 5% aqueous sodium bicarbonate (1x200mL) and brine (1x200mL). The organic solution was dried over magnesium sulfate overnight. The magnesium sulfate was removed by filtration and the solvent was evaporated to dryness under reduced pressure. The residue was dried in vacuo to obtain 5-amino-2,4,6-tribromo-3-carbamoylbenzoyl chloride (19.4 g, 89%). This compound was used in next step without purification.
- ¹³C-NMR (200MHz, DMSO-d₆):: δ= 117.53/120.39/120/70 (C-Br), 136.95 (C-COCl)), 141.65 (C-CONH₂), 144.05 (C-NH₂), 166.70 (CO in CONH₂), 167.13 (CO in COCl).

### e) 5-Acetoxyacetylamino-2,4,6-tribromo-3-carbamoylbenzoyl chloride

To a solution of 5-amino-2,4,6-tribromo-3-carbamoylbenzoyl chloride (21.76 g, 0.05 moles) **(example 11d)** in dry DMA (130 mL) at 0-5°C, acetoxyacetyl chloride (20.48 g, 0.15 moles) in dry DMA (25 mL) was added dropwise. The reaction medium was stirred at room temperature overnight. The reaction was added over ethyl acetate (250 mL) and water (400 mL). The aqueous layer was extracted with ethyl acetate (2x130 mL), the combined organic extracts were washed with brine (4x130 mL) and dried over magnesium sulfate overnight. After filtration, the solvent was removed by evaporation to dryness under reduced pressure, and dried in vacuo to obtain 5-acetoxyacetylamino-2,4,6-tribromo-3-carbamoylbenzoyl chloride (24.3 g, 91%). The product was used in the next step without purification.
- ¹H-NMR (200MHz, DMSO-d₆):: δ= 2.18 (s, 3H, CH₃), 4.69 (s, 2H, O-CH₂-CO), 6.32 (bs, 2H, NH₂), and 7.43 (m, 2H, NH₂).
- ¹³C-NMR (200MHz, DMSO-d₆):: δ= 20.53 (CH₃), 70.48 (CH₂ in O-CH₂-CO), 122.29/122.89/123.34 (C-Br), 132.65 (C-NH), 137.81 (C-COCl), 141.93 (C-CONH₂), 166.73 (CO in COCl), 167.95 (CO in CONH₂), 169.06 (CO in CONH), and 170.08 (CO in COCH₃).

### f) 5-Acetoxyacetylamino-2,4,6-tribromo-N-(2,3-dihydroxypropyl)-N-methylisophthalamide

To a solution of 5-acetoxyacetylamino-2,4,6-tribromo-3-carbamoylbenzoyl chloride (16.06 g, 0.03 moles) **(example 11e)** in acetone (250 mL), Na₂CO₃.10H₂O (9.44 g, 0.033 moles) and 3-methylamino-1,2-propanediol (3.47 g, 0.033 moles) were added. The mixture was heated at reflux for four hours. The reaction medium was cooled at room temperature and filtered. The filtrate was evaporated to dryness under reduced pressure and dried in vacuo to obtain 5-acetoxyacetylamino-2,4,6-tribromo-N-(2,3-dihydroxypropyl)-N-methylisophthalamide (17.6 g , 97%) The product was used in next step without purification.
- ¹H-NMR (200MHz, DMSO-d₆):: δ= 2.21 (s, 3H, one methyl group), 2.85/3.10 (s/s 3H, CH₃-N), 3.40-4.80 (m/m, 5H, two methylene groups and CH), 4.55 (s, 2H, O-CH₂-CO), 4.60 (m, 1H, OH), , 4.80 (m, 1H, OH), 9.70, (m, 1H, NH), and 10.20 (m, 1H, NH₂).
- ¹³C-NMR (200MHz, DMSO-d₆):: δ= 20.57 (CH₃), 34.49/37.17 (CH₃-N), 52.01/54.20 (CH₂ in CH₂NCH₃), 67.34 (CH₂ in CH₂OH), 69.90 (CH), 71.33 (O-CH₂CO), 120.62/121.48/122.09 (C-Br), 127.39/136.71/137.21 (aromatic C), 167.13 (CO in CONH₂), 168.49 (CO in CONH), 169.20 (CO in COCH₃), and 170.08 (CO in CONH).

### g) 2,4,6-Tribromo-5-hydroxyacetylamino-N-(2,3-dihydroxypropyl)-N-methylisophthalamide

To a solution of 5-acetoxyacetylamino-2,4,6-tribromo-N-(2,3-dihydroxypropyl)-N-methylisophthalamide (18.12 g, 0.030 moles) **(example 11f)** in methanol/water 1:1 (210 mL) at 50°C, a solution of 20% NaOH was added until stable pH=11. The temperature was kept for an additional hour and cooled at room temperature. The reaction medium was neutralized with hydrochloric acid/water 1:1. The solvents were evaporated to dryness under reduced pressure. The residue was purified by PLC (eluent: 5% methanol) to obtain 2,4,6-tribromo-5-hydroxyacetylamino-N-(2,3-dihydroxypropyl)-N-methylisophthalamide (12.3 g, 73%). This compound was used in next step without purification.
- ¹H-NMR (200MHz, DMSO-d₆):: δ= 2.15 (s, 3H, CH₃), 3.00/3.30 (s/s, 3H, CH₃-N), 3.40/3.80 (s/s, 5H, CH₂N/CH₂O/CH), 4.03 (s, 2H, O-CH₂-CO), 6.52 (m, 1H, NH), and 7.50 (bs, 2H, NH₂).
- ¹³C-NMR (200MHz, DMSO-d₆):: δ= 34.50/37.07 (CH₃N), 52.01 (MeN-CH₂-), 61.53 (O-CH₂-CO), 65.10 (CH₂OH), 70.17 (CH), 120.62/121.29/121.57 (C-Br), 136.60/140.01/141.18 (aromatic C), 166.66 (CO in Ar-CONH), 167.90 (CO in CONH-Ar), and 171.53 (CO in CON).

### Example 12

### Synthesis of 2,4,6-tribromo-5-hydroxyacetylamino-N-(1-hydroxymethyl-2,3-dihydroxypropyl)isophthalamide

### a) 5-Acetoxyacetylamino-2,4,6-tribromo-N-(7-hydroxy-4,4-dimethyl-3,5-dioxepinyl)isophthalamide

To a solution of 5-acetoxyacetylamino-2,4,6-tribromo-3-carbamoylbenzoyl chloride (16.06 g, 0.03 moles) **(example 11e)** in acetone (250 mL) Na₂CO₃.10H₂O (9.44 g, 0.033 moles) and 6-amino-5-hydroxy-2,2-dimethyl-1,3-dioxepine (5.32 g, 0.033 moles) were added. The mixture was heated at reflux for four hours. The reaction medium was cooled at room temperature and filtered. The filtrate was evaporated to dryness under reduced pressure and dried in vacuo to obtain 5-acetoxyacetylamino-2,4,6-tribromo-N-(7-hydroxy-4,4-dimethyl-3,5-dioxepinyl)isophthalamide (21.1 g , 97%) The product was used in next step without purification.
- ¹H-NMR (200MHz, DMSO-d₆):: δ= 1.21/1.31 (s/s, 6H, two methyl groups), 2.18 (s, 3H, CH₃-CO), 3.20-3.60 (m, 4H, 2CH₂O), 3.80 (m, 1H, CH-N), 4.15 (m, 1H, CH-OH), 4.50 (s, 2H, O-CH₂-CO), 4.60 (m, 1H, OH), 7.80/8.30 (s/s, 2H, CONH₂), 9.10 (m, 1H, NH), 9.70 (m, 1H, NH).
- ¹³C-NMR (200MHz, DMSO-d₆):: δ= 20.57 (CH₃), 23.02 (CH₃), 25.02 (CH₃), 53.21 (CH-N), 66.52/66.81 (CH₂-O), 70.19 (CH-O), 75.48 (O-CH₂-CO), 101.02 (C), 120.10/120.98/122.76 (C-Br), 127.95 (aromatic C-N), 135.05 (aromatic C-CONH), 137.31 (aromatic C-CONH₂), 166.32/167.13/168.47/169.20 (CO).

### b) 2,4,6-Tribromo-5-hydroxyacetylamino-N-(1-hydroxymethyl-2,3-dihydroxypropyl)isophthalamide

To a solution of 5-acetoxyacetylamino-2,4,6-tribromo-N-(7-hydroxy-4,4-dimethyl-3,5-dioxepinyl)isophthalamide (23.1 g, 0.035 moles) (**example 12a**) in methanol/water 1:1 (300 mL) at 50°, a solution of 20% NaOH was added until stable pH=11. The temperature was kept for an additional hour and cooled at room temperature. The reaction medium was acidulated (pH=3) with hydrochloric acid 1:1 and stirred at room temperature for an hour. Then, the reaction medium was neutralized with 5% NaOH. The solvents were evaporated to dryness under reduced pressure. The residue was purified by PLC (eluent 5% methanol) to obtain 2,4,6-tribromo-5-hydroxyacetylamino-N-(1-hydroxymethyl-2,3-dihydroxypropyl)isophthalamide.
- ¹H-NMR (200MHz, DMSO-d₆):: δ= 3.20-3-85 (m, 5H, 2CH₂-O, CH-N), 4.05 (m, 1H, CH-O), 410 (s, 2H, O-CH₂-CO), 4.60-4.70 (m, 4H, 4OH), 7.88/8.32 (s/s, 2H, CONH₂), 9.04 (m, 1H, NH), 9.30 (m, 1H, NH).
- ¹³C-NMR (200MHz, DMSO-d₆):: δ= 50.77 (CH-N), 61.08 (CH₂OH), 62.05 (O-CH₂-CO), 64.39 (CH₂OH), 70.52 (CH-OH), 120.08/121.52/124.87 (aromatic C-Br), 129.97 (aromatic C-N), 135.08 (aromatic C-CONH), 138.34 (aromatic C-CONH₂), 166.22/167.13/168.98 (CO groups).

### Example 13

### Synthesis of 2,4,6-tribromo-5-hydroxyacetylamino-N,N,N', N'-tetrakis-(2-hydroxyethyl)isophthalamide

To a solution of 5-acetoxyacetylamino-2,4,6-tribromoisophthaloyl dichloride (19.4 g, 0.035 moles) (**example 5c**) in acetone (300 mL), sodium carbonate decahydrate (22.03 g, 0.077 moles) and bis-(2-hydroxyethyl)amine (8.09 g, 0.077 moles) in acetone acetone (10 mL) were added. The mixture was heated at solvent reflux for three hours (the process was followed by TLC: acetone/n-hexane 3:2). The reaction medium was filtered and the solvent was removed by evaporation to dryness under reduced pressure. The crude, 5-acetoxyacetylamino-2,4,6-tribromo-N,N,N',N'-tetrakis-(2-hydroxyethyl)isophthalamide, (19.75 g, 89%), was dissolved in methanol/water 1:1 (200 mL) at 50°C. A solution of 20% NaOH was added until stable pH = 11. The temperature was kept for an additional hour and cooled at room temperature. The reaction medium was neutralized with hydrochloric acid/water 1:1. The solvents were evaporated to dryness under reduced pressure. The residue was purified by PLC (eluent: 8% methanol) to obtain 5-hydroxyacetylamino-2,4,6-tribromo-N,N,N',N'-tetrakis-(2-hydroxyethyl)isophthalamide (17 g, global yield=75%).
- ¹H-NMR (200MHz, DMSO-d₆):: δ= 3.31 (m, 4H, 2CH₂-N), 3.75 (m, 12H, 4CH₂-OH/2CH₂-N), 4.08 (s, 2H, O-CH₂-CO), 4.51 (bs, 4H, 4OH), 4.65 (bs, 1H, OH), 10.07 (m, 1H, NH).
- ¹³C-NMR (200MHz, DMSO-d₆):: δ= 49.51/55.68 (CH₂-N), 59.70 (CH₂OH), 118.96/120.53/120.91 (C-Br), 127.64 (C-NH), 133.82/134.12 (C-CON), 170.21 (CO), 170.45 (CO).

### Example 14

### Synthesis of 2,4,6-tribromo-5-[N-(2-hydroxyethyl)hydroxyacetylamino]-N-(1-hydroxymethyl-2,3-dihydroxypropyl)isophthalamide

To a solution of 5-acetoxyacetylamino-2,4,6-tribromo-N-(7-hydroxy-4,4-dimethyl-3,5-dioxepinyl)isophthalamide **(example 12a)** (20 g, 0.03 moles) in methanol (150 mL), calcium chloride (3.36 g, 0.03 moles), NaOH (1.80 g, 0.045 moles), and 2-chloroethanol (3.64 g, 0.045 moles) were added. The reaction medium was heated at solvent reflux for four hours. Then, the reaction medium was cooled at room temperature and filtered. The filtrate was evaporated to dryness under reduced pressure and dried in vacuo to obtain 5-[N-(2-hydroxyethyl)acetoxyacetylamino-2,4,6-tribromo-N-(7-hydroxy-4,4-dimethyl-3,5-dioxepinyl)isophthalamide (19 g).

This crude was dissolved in methanol/water 1:1 (250 mL) and heated at 50°, then a solution of 20% NaOH was added until stable pH=11. The temperature was kept for an additional hour and cooled at room temperature. The reaction medium was acidulated (pH=3) with hydrochloric acid 1:1 and stirred at room temperature for an hour. Then, the reaction medium was neutralized with 5% NaOH. The solvents were evaporated to dryness under reduced pressure. The residue was purified by PLC (eluent 5% methanol) to obtain 2,4,6-tribromo-5-[N-(2-hydroxyethyl)hydroxyacetylamino]-N-(1-hydroxymethyl-2,3-dihydroxypropyl)isophthalamide (11.7 g, 62%).
- ¹H-NMR (200 MHz, DMSO-d₆):: δ= 3.33-3-90 (m, 9H, 3CH₂-O, CH₂-N CH-N), 4.00 (m, 1H, CH-O), 4.08 (s, 2H, O-CH₂-CO), 4.53-4.68 (m, 5H, 5OH), 7.91/8.42 (s/s, 2H, CONH₂), 8.99 (m, 1H, NH), 9.20 (m, 1H, NH).
- ¹³C-NMR (200 MHz, DMSO-d₆):: δ= 50.64 (CH₂-N), 58.30 (CH₂-O), 60.84 (CH₂-O), 61.10 (O-CH₂-CO), 64.30 (CH₂-O), 70.52 (CH-O), 122.12/127.58/128.25 (aromatic C-BR), 138.40 (aromatic C-CONH), 139.43 (aromatic C-CONH2), 145.73 (aromatic C-N), 166.22/167.13/170.98 (CO groups).

### Example 15

### Synthesis of 2,4,6-tribromo-5-hydroxyacetylamino-N',N'-bis-(2,3-dihydroxypropyl)-N, N'-dimethylisophthalamide

To a cooled (0-5°C) mixture of 5-acetoxyacetylamino-2,4,6-tribromoisophthaloyl dichloride (**example 5c**) (72 g, 0.129 moles) and sodium carbonate decahydrate (81.5 g, 0.285 moles) in acetone (720 mL) was added dropwise a solution of 3-methylamino-1,2-propanediol (29.2 g, 0.285 moles) in acetone (60 mL) over 35-minute period. When the addition was complete the reaction mixture was boiled for 4 hours.

The mineral salts were removed by filtration. The acetone was evaporated under reduced pressure, the residue dissolved in methanol (144 mL) and precipitated by adding into 2-propanol (1152 mL). The resulting precipitate was collected by filtration, washed and dried in vacuo.

The collected precipitate was redissolved in 1:1 water/methanol (420 mL) and the pH adjusted to approximately 11 with 5 % sodium hydroxide. The solution was heated at 40 °C for two hours and then chilled and neutralized (pH 5-6) with concentrated hydrochloric acid.

The resulting solution was concentrated to dryness and the residue redissolved in methanol to remove mineral salts by filtration. The methanol was evaporated yielding crude 2,4,6-tribromo-5-hydroxyacetylamino-N', N'-bis-(2,3-dihydroxypropyl)-N, N'-dimethylisophthalamide (72.3 g).

The crude product was purified by reverse-phase preparative liquid chromatography. After evaporation and drying in vacuo, 67.1 g of compound were obtained in an overall (reaction-purification) yield of 80.0 %.
- ¹H-NMR (200 MHz, DMSO-d₆):: δ= 9.81 (broadened signal, 1H, exchangeable with D₂O, NH-Ph), 5.86 (broadened signal, exchangeable with D₂O, 1H, OH), 4.85 (broadened signal, exchangeable with D₂O, 2H, 2OH), 4.65 (broadened signal, exchangeable with D₂O, 2H, 2OH), 4.03 (s, 2H, HOCH₂CO), 3.84 (m, 2H), 3.65 (m, 4H), 3.40-3.20 (m, 4H), 3.15 and 2.85 (2s, 6H, N-CH₃).
- ¹³C-NMR (200 MHz, DMSO-d₆):: δ= 171.3 (CO), 165.8 (CO), 165.9 (CO), 140.4/140.1 (C-CON), 137.2 (C-NH), 122.6/121.7/115.0 (C-Br), 69.9 (CH), 64.1 (CH₂), 61.5 (CH₂), 50.3 (CH₂), 37.2 (N-CH₃).

### Example 16

### Synthesis of 2,4,6-tribromo-5-methoxyacetylamino-N,N'-bis-(2,3-dihydroxypropyl)-N, N'-dimethylisophthalamide

### a) 2,4,6-tribromo-5-methoxyacetylaminoisophthaloyl dichloride

To a solution of 5-amino-2,4,6-tribromoisophthaloyl dichloride (63 g, 0.138 moles) (**example 5b**) in dry DMA (380 mL) at 0-5°C, methoxyacetyl chloride (44.9 g, 0.414 moles) in dry DMA (45 mL) was added dropwise. The reaction mixture was stirred at room temperature overnight.

The reaction was added over ethyl acetate (400 mL) and water (400 mL). The ethyl acetate layer was separated, washed with 5 % NaHCO₃ solution (3 x 350 mL), water (350 mL), dried over Na₂SO₄ and evaporated under pressure to give 2,4,6-tribromo-5-methoxyacetylisophthaloyl dichloride (68.9 g, 94.8 %). This product was used as such in the next step.
- ¹H-NMR (200 MHz, DMSO-d₆):: δ= 10.3 (2s, 1H, exchangeable with D₂O, NH-Ph), 4.01 (s, 2H, MeOCH₂), 3.46 (s, 3H, CH₃O).
- ¹³C-NMR (200 MHz, DMSO-d₆):: δ= 169.2 (CO), 166.7 (CO) 141.2, 135.9, 123.9 (C-Br), 121.2 (C-Br), 74.2 (MeOCH₂), 59.3 (CH₃), 49.0 (CH₂).

### b) 2,4,6-tribromo-5-methoxyacetylamino-N, N'-bis-(2,3-dihydroxypropyl)-N, N'-dimethylisophthalamide

To a cooled (0-5°C) mixture of 2,4,6-tribromo-5-methoxyacetylisophthaloyl dichloride (**example 16a**) (65 g, 0.123 moles) and sodium carbonate decahydrate (77.6 g, 0.271 moles) in acetone (650 mL) was added dropwise a solution of 3-methylamino-1,2-propanediol (28.5 g, 0.271 moles) in acetone (60 mL) over 35-minute period. When the addition was complete the reaction mixture was boiled for 4 hours.

The mineral salts were removed by filtration. The acetone was evaporated under reduced pressure and the residue dissolved in methanol (130 mL) and precipitated by adding into 2-propanol (1040 mL). The resulting precipitate was collected by filtration, washed and dried in vacuo.

The crude product was purified by reverse-phase preparative liquid chromatography. After evaporation and drying in vacuo, 69.8 g of 2,4,6-tribromo-5-methoxyacetylamino-N,N'-bis-(2,3-dihydroxypropyl)-N,N'-dimethylisophthalamide were obtained in an overall (reaction-purification) yield of 85.5 %.
- ¹H-NMR (200 MHz, DMSO-d₆):: δ= 10.3 (broadened signal, 1H, exchangeable with D₂O, NH-Ph), 5.2-4.2 (broadened signal, exchangeable with D₂O, 4H, OH), 4.01 (s, 2H, MeOCH₂), 3.91-3.21 (m, 10H), 3.46 (s, 3H, CH₃O), 3.15 and 2.84 (2s, 6H, N-CH₃)
- ¹³C-NMR (200 MHz, DMSO-d₆):: δ= 169.4 (CO), 169.2 (CO) 135.7, 125.7, 120.8 (C-Br), 120.5 (C-Br), 74.1 (MeOCH₂), 69.4 (CH), 64.05 (CH₂), 59.3 (CH₃), 49.0 (CH₂), 34.1 and 28.8 (N-CH₃)

### Example 17

### Synthesis of 2,4,6-tribromo-5-hydroxyacetylamino-N-(2,3-dihydroxypropyl)-N'-(1,3-dihydroxy-2-propyl)isophthalamide

To a solution of 5-acetoxyacetylamino-2,4,6-tribromo-3-[N-(2,3-diacetoxypropyl)carbamoyl]benzoyl chloride (25 g, 0.036 moles) **(example 4f)** in acetone (270 mL), Na₂CO₃.10H₂O (11.44 g, 0.040 moles) and 2-amino-1,3-propanediol (4.20 g, 0.040 moles) were added. The mixture was heated at reflux for four hours. The reaction medium was cooled at room temperature and evaporated to dryness under reduced pressure. The residue was treated with methanol (50 ml) and filtrated. The alcoholic solution was evaporated to dryness under reduced pressure (30 g).
To a solution of the last crude in methanol/water 1:1 (250 mL) at 50°C, a solution of 20% NaOH was added until stable pH=11. The temperature was kept for an additional hour and cooled at room temperature. The reaction medium was neutralized with hydrochloric acid/water 1:1. The solvents were evaporated to dryness under reduced pressure. The residue was purified by PLC (eluent: 5% methanol) to obtain 2,4,6-tribromo-5-hydroxyacetylamino-N-(2,3-dihydroxypropyl)-N'-(1,3-dihydroxy-2-propyl)isophthalamide(20 g, 75%).
- ¹H-NMR (200 MHz, DMSO-d₆):: δ=3.00-3.90 (m, 10H, four methylene groups and two CH groups), 4.00 (s, 2H, OCH2-CO), 4.60 (m, 2H, two hydroxyl groups), 4.80 (m, 2H, two hydroxyl groups), 5.81 (m, 1H, OH), 8.80 (m, 2H, 2NH), and 9.80 (m, 1H, NH).
- ¹³C-NMR (200 MHz, DMSO-d₆):: δ= 41.90 (CH₂ in NH-CH₂-), 52.38 (CH in CH(CH₂)CH₂-), 61.62 (CH₂OH), 63.40/63.90 (CH₂OH), 64.10 (O-CH₂-CO), 70.17 (CH), 115.82/120.81/122.43 (C-Br), 136.94/140.31/141.95 (aromatic C), 165.86/166.07 (CO in Ar-CONH), 166.71 (CO in CONH-Ar).

### Example 18

### Synthesis of 2,4,6-tribromo-5-[N-(2-hydroxyethyl)acetylamino]-N-(1,3-dihydroxy-2-propyl)isophthalamide

### a) 5-Acetylamino-2,4,6-tribromo-3-carbamoylbenzoyl chloride

To a solution of 5-amino-2,4,6-tribromo-3-carbamoylbenzoyl chloride (50.5 g, 0.116 moles) **(example 11d )** in dry DMA (260 mL) at 0-5°C, acetyl chloride (24.7 ml, 0.348 moles) in dry DMA (25 mL) was added dropwise. The reaction medium was stirred at room temperature overnight. The reaction was added over ethyl acetate (500 mL) and water (400 mL). The aqueous layer was extracted with ethyl acetate (250 mL), the combined organic extracts were washed with brine (3x250 mL) and dried over magnesium sulfate overnight. After filtration, the solvent was removed by evaporation to dryness under reduced pressure, and dried in vacuo to obtain 5-acetylamino-2,4,6-tribromo-3-carbamoylbenzoyl chloride (52 g, 94 %). The product was used in the next step without further purification.
- ¹H-NMR (200 MHz, DMSO-d₆):: δ= 9.9 (2s, 1H, exchangeable with D₂O, CONH-Ph), 7.91 and 7.80 (2s, 2H, exchangeable with D₂O, CONH₂), 2.20 (s, 3H, COCH₃).
- ¹³C-NMR (200 MHz, DMSO-d₆):: δ= 167.2 (CO), 167.3 (CO), 166.7 (CO), 141.2, 136.7, 131.9, 122.5 (C-Br), 122.3 (C-Br), 122.2 (C-Br), 24.1 (CH₃).

### b) 5-Acetylamino-2,4,6-tribromo-N-(1,3-dihydroxy-2-propyl)isophthalamide

To a solution of 5-acetylamino-2,4,6-tribromo-3-carbamoylbenzoyl chloride (45 g, 0.094 moles) **(example 18a)** in acetone (450 mL), Na₂CO₃.10H₂O (32.3 g, 0.113 moles) and 2-amino-1,3-propanediol (10.3 g, 0.113 moles) were added. The mixture was heated at reflux for four hours. The reaction medium was cooled at room temperature and filtered. The filtrate was evaporated to dryness under reduced pressure and dried in vacuo to obtain 5-acetylamino-2,4,6-tribromo-N-(1,3-dihydroxy-2-propyl)isophthalamide (46 g , 92 %) The product was used in next step without further purification.
- ¹H-NMR (200 MHz, DMSO-d₆):: δ= 9.9 (2s, 1H, exchangeable with D₂O, CONH-Ph), 8.1 (broad signal, 1H, exchangeable with D₂O, Ph-CONH), 7.9 and 7.8 (2s, 2H, exchangeable with D₂O, CONH₂), 5.5-5.1 (2m, 2H, exchangeable with D₂O exchangeable with D₂O, OH), 4.8 (m, 1H, CH), 3.68-3.51 (m, 4H, CH₂OH), 2.15 (s, 3H, COCH₃).
- ¹³C-NMR (200 MHz, DMSO-d₆):: δ= 167.2 (CO), 167.1 (CO), 163.7 (CO), 136.8, 134.4, 127.5, 121.0 (C-Br), 120.1 (C-Br), 119.1 (C-Br), 63.5 (CH₂), 55.3 (CH), 24.1 (CH₃).

### c) 5-Acetylamino-2,4,6-tribromo-N-(1,3-diacetoxy-2-propyl)isophthalamide

To a solution of 5-acetylamino-2,4,6-tribromo-N-(1,3-dihydroxy-2-propyl)isophthalamide (45 g, 0.084 moles) **(example 18b)** in ethyl acetate (400 mL), dimethylaminopyridine (513 mg) was added with stirring. The mixture was heated at solvent reflux for ten minutes. Then acetic anhydride (31.7 mL) was added. The heating was continued for four hours. Ethanol (20 mL) was added and the reflux continued for half hour. The solvent was removed by evaporation to dryness under reduced pressure. The residue was purified by precipitation in methanol/ethyl ether. The resulting solid was filtered, and dried in vacuo to obtain 5-acetylamino-2,4,6-tribromo-N-(1,3-diacetoxy-2-propyl)isophthalamide (50.6 g, 98 %). The product was used in next step without further purification.
- ¹H-NMR (200 MHz, DMSO-d₆):: δ= 10.21 (2s, 1H, exchangeable with D₂O, CONH-Ph), 8.1 (broad signal, 1H, exchangeable with D₂O, Ph-CONH), 8.2 and 7.9 (2s, 2H, exchangeable with D₂O, CONH₂), 4.4 (m, 1H, CH), 4.07-3.90 (m, 4H, CH₂OAc), 2.20 (s, 3H, COCH₃), 2.06 and 2.03 (2s, 6H, COCH₃).
- ¹³C-NMR (200 MHz, DMSO-d₆):: δ= 170.8 (CO), 167.5 (CO), 167.3 (CO), 163.7 (CO), 136.7, 135.1, 127.5, 121.1 (C-Br), 120.3 (C-Br), 119.8 (C-Br), 63.9 (CH₂), 54.3 (CH), 24.2 (CH₃), 20.7 (CH₃), 20.5 (CH₃).

### d) 2,4,6-Tribromo-5-[N-(2-hydroxyethyl)acetylamino]-N-(1,3-dihydroxy-2-propyl)isophthalamide

To a suspension of 5-acetylamino-2,4,6-tribromo-N-(1,3-diacetoxy-2-propyl)isophthalamide **(example 18c)** (50g, 0.094 moles) and anhydrous potassium carbonate (19.8 g , 0.187 moles) in methanol (350 mL) 2-chloroethanol (13.4 mL, 0.187 moles) was added all at once and the mixture was stirred and kept at 40°C for 4 hours and then left at room temperature overnight.

The mineral salts were removed by filtration. The methanol was evaporated under reduce pressure and to the resulting residue dichloromethane (400 mL) and water (400 mL) were added. The dichloromethane layer was separated, washed with 5 % NaHCO₃ solution (3 x 200 mL), water (200 mL), dried over Na₂SO₄ and evaporated under reduce pressure.

The resulting residue (53.2 g) was dissolved in 1:1 water/methanol (320 mL) and the pH adjusted to approximately 11 with 5 % sodium hydroxide. The solution was heated at 40°C for two hours and then chilled and neutralized (pH 5-6) with concentrated hydrochloric acid.

The resulting solution was concentrated to dryness, the residue redissolved in methanol and mineral salts removed by filtration. The methanol was evaporated yielding crude 2,4,6-tribromo-5-[N-(2-hydroxyethyl)acetylamino]-N-(1,3-dihydroxy-2-propyl)isophthalamide (48.6 g).

The crude product was purified by reverse-phase preparative liquid chromatography. After evaporation and drying in vacuo, 41.3 g of 2,4,6-tribromo-5-[N-(2-hydroxyethyl)acetylamino]-N-(1,3-dihydroxy-2-propyl)isophthalamide were obtained in an overall (reaction-purification) yield of 76.3 %.
- ¹H-NMR (200 Mz, DMSO-d₆):: δ= 8.2 (broad signal, 1H, exchangeable with D₂O, Ph-CONH), 8.2 and 7.9 (2s, 2H, exchangeable with D₂O, CONH₂), 5.5-4.8 (3m, 3H, OH), 4.05 (m, 1H, CH), 3.7-3.5 (m, 8H), 2.15 (s, 3H, COCH₃).
- ¹³C-NMR (200 Mz, DMSO-d₆):: δ= 168.1 (CO), 167.7 (CO), 162.3 (CO), 145.7, 139.7, 137.1, 127.8 (C-Br), 126.8 (C-Br), 121.8 (C.Br), 63.5 (CH₂), 63.4 (CH₂), 58.2 (CH₂), 55.3 (CH), 47.7 (CH₂), 22.4 (CH₃)

### Example 19

### Synthesis of 2,4,6-tribromo-5-[N-(2-hydroxyethyl)acetylamino]-N-(1-hydroxymethyl-2,3-dihydroxypropyl)isophthalamide

### a) 5-Acetylamino-2,4,6-tribromo-N-(7-hydroxy-4,4-dimethyl-3,5-dioxepinyl)isophthalamide

To a solution of 5-acetylamino-2,4,6-tribromo-3-carbamoylbenzoyl chloride (51 g, 0.117 moles) **(example 18a)** in acetone (500 mL), Na₂CO₃.10H₂O (40 g, 0.14 moles) and 6-amino-5-hydroxy-2,2-dimethyl-1,3-dioxepine (22.6 g, 0.14 moles) were added. The mixture was heated at reflux for four hours. The reaction medium was cooled at room temperature and filtered. The filtrate was evaporated to dryness under reduced pressure and dried in vacuo to obtain 5-acetylamino-2,4,6-tribromo-N-(7-hydroxy-4,4-dimethyl-3,5-dioxepinyl)isophthalamide (57.7 g , 82 %) The product was used in next step without further purification.
- ¹H-NMR (200 MHz, DMSO-d₆):: δ= 9.8 (2s, 1H, exchangeable with D₂O, CONH-Ph), 8.1 (broad signal, 1H, exchangeable with D₂O, Ph-CONH), 8.0 and 7.9 (2s, 2H, exchangeable with D₂O, CONH₂), 4.9 (d, 1H, exchangeable with D₂O exchangeable with D₂O, OH), 4.2-3.2 (m, 6H), 2.20 (s, 3H, COCH₃), 1.2 (s, 6H, CH₃).
- ¹³C-NMR (200 MHz, DMSO-d₆):: δ= 167.3 (CO), 167.1 (CO), 165.8 (CO), 136.6, 135.4, 127.3, 121.0 (C-Br), 120.3 (C-Br), 120.1 (C-Br), 100.64 (C), 70.2 (CH), 62.4 (CH₂), 59.2 (CH₂), 54.8 (CH), 24.6 (CH₃), 24.4 (CH₃), 24.1 (COCH₃).

### b) 2,4,6-Tribromo-5-[N-(2-hydroxyethyl)acetylamino]-N-(1-hydroxymethyl-2,3-dihydroxypropyl)isophthalamide

To a suspension of 5-acetylamino-2,4,6-tribromo-N-(7-hydroxy-4,4-dimethyl-3,5-dioxepinyl)isophthalamide **(example 19a)** (55 g, 0.091 moles) and anhydrous potassium carbonate (19.3 g , 0.182 moles) in methanol (400 mL) 2-chloroethanol (12.2 mL, 0.182 moles) was added all at once and the mixture was stirred and kept at 40°C for 4 hours and then left at room temperature overnight.

The mineral salts were removed by filtration. The methanol was evaporated under reduce pressure and to the resulting residue dichloromethane (400 mL) and water (400 mL) were added. The dichloromethane layer was separated, washed with 5 % NaHCO₃ solution (3 x 200 mL), water (200 mL), dried over Na₂SO₄ and evaporated under reduce pressure.

The resulting residue (54.8 g) was dissolved in 1:1 water/methanol (320 mL) and the pH adjusted to approximately 1 with 35 % hydrochloric acid The solution was stirred at room temperature for two hours and then neutralized (pH 5-6) with 20% sodium hydroxide.

The resulting solution was concentrated to dryness, the residue redissolved in methanol and mineral salts removed by filtration. The methanol was evaporated yielding crude 2,4,6-tribromo-5-[N-(2-hydroxyethyl)acetylamino]-N-(1-hydroxymethyl-2,3-dihydroxypropyl)isophthalamide (47.5 g).

The crude product was purified by reverse-phase preparative liquid chromatography. After evaporation and drying in vacuo, 41.3 g of 2,4,6-tribromo-5-[N-(2-hydroxyethyl)acetylamino)-N-(1-hydroxymethyl-2,3-dihydroxypropyl)isophthalamide were obtained in an overall (reaction-purification) yield of 75 %.
- ¹H-NMR (200 Mz, DMSO-d₆):: δ= 8.2 (broad signal, 1H, exchangeable with D₂O, Ph-CONH), 8.1 and 7.9 (2s, 2H, exchangeable with D₂O, CONH₂), 5.3-4.9 (4m, 4H, OH), 4.2-3.2 (m, 10H), 2.15 (s, 3H, COCH₃).
- ¹³C-NMR (200 Mz, DMSO-d₆):: δ= 168.1 (CO), 167.7 (CO), 162.3 (CO), 145.7, 139.7, 138.3, 127.8 (C-Br), 127.1 (C-Br), 122.1 (C-Br), 70.5 (CH), 64.4 (CH₂), 60.8 (CH₂), 58.3 (CH₂), 53.7 (CH), 47.6 (CH₂), 22.4 (COCH₃).

### Example 20

### Synthesis of N,N'-bis-[2,4,6-tribromo-3-[N-(2,3-dihydroxypropyl)-N-methylcarbamoyl]-5-[N-(2,3-dihydroxypropyl)carbamoyl]phenyl]diglycolamide

### a) N,N'-bis-[2,4,6-tribromo-3-chlorocarbonyl-5-[N-(2,3diacetoxypropyl)carbamoyl]phenyl]diglycolamide

To a stirred and heated (reflux) solution of 5-amino-2,4,6-tribromo-3-[N-(2,3-diacetoxypropyl)carbamoyl]benzoyl chloride **(example 4e)** (42 g, 0.07 moles) in dioxane was added distilled diglycolyl chloride (6.6 g, 0.0389 moles) over a twenty-minute period. When the addition is complete, the reaction mixture was left under reflux for two hours.

The reaction mixture was chilled and the resulting precipitate filtered off, washed with dioxane and dried to obtain N,N'-bis-[2,4,6-tribromo-3-chlorocarbonyl-5-[N-(2,3-diacetoxypropyl)carbamoyl]phenyl]diglycolamide (36.3 g, 80.6 %). The product was used in the next step without further purification.
- ¹H-NMR (200 MHz, DMSO-d₆):: δ= 9.9 (2s, 2H, exchangeable with D₂O, NH-Ph), 8.6 (broad signal, 2H, exchangeable with D₂O, 2H, CO-NH), 5.20 (m, 2H, CH), 4.13 (m, 4H, CH₂), 3.99 (s, 4H, COCH₂OCH₂CO) 3.50 (m, 4H, CH₂), 2.18 (s, 6H, COCH₃), 2.05 (s, 6H, COCH₃).
- ¹³C-NMR (200 MHz, DMSO-d₆):: δ= 173.7 (CO), 172.0 (CO), 170.3 (CO), 166.7 (CO), 165.0 (CO), 141.2, 136.5, 131.6, 122.9 (C-Br), 122.8 (C-Br), 122.5 (C-Br), 68.3 (CH), 62.4 (CH₂), 61.9 (CH₂), 39.7 (CH₂), 24.1 (CH₃), 20.5 (CH₃).

### b) N,N'-bis-[2,4,6-tribromo-3-[N-(2,3-dihydroxypropyl)-N-methylcarbamoyl]-5-[N-(2,3-dihydroxypropyl)carbamoyl]-phenyl]diglycolamide,

Into a cooled (0-5°C) mixture of N,N'-bis-[2,4,6-tribromo-3-chlorocarbonyl-5-[N-(2,3-diacetoxypropyl)carbamoyl]phenyl]diglycolamide **(example 20a)** (82 g, 0.064 moles) and sodium carbonate decahydrate (54.9 g, 0.192 moles) in DMF (246 mL) was added dropwise a solution of 3-methylamino-1,2-propanediol (16.8 g, 0.16 moles) in DMF (42 mL) over 25-minute period. When the addition was complete the reaction mixture was allowed to warm to room temperature and stirred for 24 hours.

The mineral salts were removed by filtration. The DMF was evaporated under reduced pressure and the residue dissolved in methanol (120 mL) and precipitated by adding into 2-propanol (960 mL). The resulting precipitate was collected by filtration, washed and dried in vacuo.

The collected precipitate was redissolved in 1:1 water/methanol (320 mL) and the pH adjusted to approximately 11 with 5 % sodium hydroxide. The solution was heated at 40 °C for two hours and then chilled and neutralized (pH 5-6) with concentrated hydrochloric acid.

The resulting solution was concentrated to dryness and the residue redissolved in methanol to remove mineral salts by filtration. The methanol was evaporated yielding crude N,N'-bis-[2,4,6-tribromo-3-[N-(2,3-dihydroxypropyl)-N-methylcarbamoyl]-5-[N-(2,3-dihydroxypropyl)carbamoyl]phenyl]diglycolamide (73 g).

The crude product was purified by reverse-phase preparative liquid chromatography. After evaporation and drying in vacuo, 65 g of N,N'-bis-[2,4,6-tribromo-3-[N-(2,3-dihydroxypropyl)-N-methylcarbamoyl]-5-[N-(2,3-dihydroxypropyl)carbamoyl]phenyl]diglycolamide were obtained in an overall (reaction-purification) yield of 81.0 %.
- ¹H-NMR (200 MHz, DMSO-d₆):: δ= 9.9 (2s, 2H, exchangeable with D₂O, NH-Ph), 8.7 (broad signal, 2H, exchangeable with D₂O, 2H, CO-NH), 5.41 (m, 2H, OH), 5.18 (m, 2H, OH), 4.51 (m, 2H, OH), 4.47 (m, 2H, OH), 3.94 (s, 4H, COCH₂OCH₂CO), 3.85-3.21 (m, 20H), 3.21 and 2.85 (2s, 6H, CH₃).
- ¹³C-NMR (200 MHz, DMSO-d₆):: δ= 1169.4(CO), 166.5(CO), 166.3(CO), 136.0, 135.8, 122.0, 120.8(C-Br), 120.7(C-Br), 120.4(C-Br), 71.0 (CH), 69.4 (CH), 67.0 (CH₂), 66.5 (CH₂), 63.4 (CH₂), 49.0 (CH₂), 41.9 (CH₂), 31.2 and 27.8(CH₃).

### Example 21

### Synthesis of N,N'-bis-[2,4,6-tribromo-3-[N-(2,3-dihydroxypropyl)-N-methylcarbamoyl]-5-[N-(2,3-dihydroxypropyl)carbamoyl]phenyl]malonamide

### a) N,N'-bis-[2,4,6-tribromo-3-chlorocarbonyl-5-[N-(2,3-diacetoxypropyl)carbamoyl]phenyl]malonamide

To a stirred and refluxed solution of 5-amino-2,4,6-tribromo-3-[N-(2,3-diacetoxypropyl)carbamoyl]benzoyl chloride **(example 4e)** (58 g, 0.0977 moles) in dioxane was added distilled malonyl chloride (7.5 g, 0.053 moles) over a thirty-minute period. When the addition is complete, the reaction mixture was left under reflux for two hours.

The reaction mixture was chilled and the resulting precipitate filtered off, washed with dioxane and dried to obtain N,N'-bis-[2,4,6-tribromo-3-chlorocarbonyl-5-[N-(2,3-diacetoxypropyl)carbamoyl]phenyl]malonamide (56 g, 91.3 %). The product was used in the next step without further purification.
- ¹H-NMR (200 MHz, DMSO-d₆):: δ= 9.8 (2s, 2H, exchangeable with D₂O, NH-Ph), 8.5 (broad signal, 2H, exchangeable with D₂O, 2H, CO-NH), 5.30 (m, 2H, CH), 4.21 (m, 4H, CH₂), 3.65 (m, 4H, CH₂), 3.57 (s, 2H, COCH₂CO), 2.13 (s, 6H, COCH₃), 2.05 (s, 6H, COCH₃).
- ¹³C-NMR (200 MHz, DMSO-d₆):: δ= 173.7 (CO), 172.0 (CO), 170.3 (CO), 166.7 (CO), 165.0 (CO), 141.2, 136.5, 131.6, 122.9 (C-Br), 122.8 (C-Br), 122.5 (C-Br), 68.3 (CH), 62.4 (CH₂), 45.9 (CH₂, 39.7 (CH₂), 24.1 (CH₃), 20.5 (CH₃).

### b) N,N'-bis-[2,4,6-tribromo-3-[N-(2,3-dihydroxypropyl)-N-methylcarbamoyl]-5-[N-(2,3-dihydroxypropyl)carbamoyl]phenyl]malonamide

Into a cooled (0-5°C) mixture of N,N'-bis-[2,4,6-tribromo-3-chlorocarbonyl-5-[N-(2,3-diacetoxypropyl)carbamoyl]phenyl]malonamide **(example 21a)** (55 g, 0.044 moles) and sodium carbonate decahydrate (37.7 g, 0.132moles) in DMF (165 mL) was added dropwise a solution of 3-methylamino-1,2-propanediol (11.5 g, 0.11 moles) in DMF (28 mL) over 20-minute period. When the addition was complete the reaction mixture was allowed to reach room temperature and stirred for 24 hours.

The mineral salts were removed by filtration. The DMF was evaporated under reduced pressure and the residue dissolved in methanol (110 mL) and precipitated by adding into 2-propanol (880 mL). The resulting precipitate was collected by filtration, washed and dried in vacuo.

The collected precipitate was redissolved in 1:1 water/methanol (220 mL) and the pH adjusted to approximately 11 with 5 % sodium hydroxide. The solution was heated at 40 °C for two hours and then chilled and neutralized (pH 5-6) with concentrated hydrochloric acid.
The resulting solution was concentrated to dryness and the residue redissolved in methanol to remove mineral salts by filtration. The methanol was evaporated yielding crude N,N'-bis-[2,4,6-tribromo-3-[N-(2,3-dihydroxypropyl)-N-methylcarbamoyl]-5-[N-(2,3-dihydroxypropyl)carbamoyl]phenyl]malonamide (49 g).

The crude product was purified by reverse-phase preparative liquid chromatography. After evaporation and drying in vacuo, 45 g of N,N'-bis-[2,4,6-tribromo-3-[N-(2,3-dihydroxypropyl)-N-methylcarbamoyl]-5-[N-(2,3-dihydroxypropyl)carbamoyl]phenyl]malonamide were obtained in an overall (reaction-purification) yield of 83.6 %.
- ¹H-NMR (200 MHz, DMSO-d₆):: δ= 9.4 (2s, 2H, exchangeable with D₂O, NH-Ph), 8.51 (broad signal, 2H, exchangeable with D₂O, 2H, CO-NH), 5.34 (m, 2H, OH), 5.12 (m, 2H, OH), 4.51 (m, 2H, OH), 4.47 (m, 2H, OH), 3.85-3.02 (m, 20H), 3.51 (s, 2H, COCH₂CO), 3.18 and 2.68 (2s, 6H, CH₃).
- ¹³C-NMR (200 MHz, DMSO-d₆):: δ= 173.7 (CO), 169.8 (CO), 167.1 (CO), 136.0, 135.9, 126.4, 120.9 (C-Br), 120.8 (C-Br), 120.4 (C-Br), 71.4 (CH), 68.6 (CH), 67.5 (CH₂), 64.8 (CH₂), 49.2 (CH₂), 45.9 (CH₂), 41.8 (CH₂), 32.9 and 29.2 (CONCH₃).

### Example 22

### Synthesis of N,N'-bis-[2,4,6-tribromo-3-[N-(2,3-dihydroxypropyl)-N-methylcarbamoyl]-5-[N-(2,3-dihydroxypropyl)carbamoyl]phenyl]-N,N'-dimethylmalonamide

### a) N,N'-bis-[2,4,6-tribromo-3-[N-(2,3-diacetoxypropyl)-N-methylcarbamoyl]-5-[N-(2,3-diacetoxypropyl)carbamoyl]phenyl]malonamide

Into a cooled (0-5°C) mixture of N,N'-bis-[2,4,6-tribromo-3-chlorocarbonyl-5-[N-(2,3-diacetoxypropyl)carbamoyl]phenyl]malonamide **(example 21a)** (68 g, 0.054 moles) and sodium carbonate decahydrate (46.3 g, 0.162 moles) in DMF (204 mL) was added dropwise a solution of 3-methylamino-1,2-propanediol (14.2 g, 0.135 moles) in DMF (37 mL) over 25-minute period. When the addition was complete, the reaction mixture was allowed to reach room temperature and stirred for 24 hours.

The mineral salts were removed by filtration. The DMF was evaporated under reduced pressure and the residue dissolved in methanol (136 mL) and precipitated by pouring into 2-propanol (1.1 L), The resulting precipitate was collected by filtration, washed and dried in vacuo.

The collected precipitate was dissolved in DMA (136 mL) and 4-dimethylaminopyridina (0.33 g, 0.027 moles) added. An ice bath was applied, and acetic anhydride (30.2 mL, 0.32 moles) was added at such a rate as to maintain the temperature below 10°C. The ice bath was removed and the solution was stirred at 45°C for 16 hours.
After this period, water (500 mL) was added to the mixture reaction and stirred for 30 minutes. The mixture was extracted with dichloromethane (300 mL). The dichloromethane layer was separated, washed with 5 % NaHCO₃ solution (3 x 200 mL), water (200 mL), dried over Na₂SO₄ and evaporated under pressure to give N,N'-bis-[2,4,6-tribromo-3-[N-(2,3-diacetoxypropyl)-N-methylcarbamoyl]-5-[N-(2,3-(diacetoxypropyl)carbamoyl]phenyl]malonamide (71.3 g, 84.6 %). This product was used as such in the next step.
- ¹H-NMR (200 MHz, DMSO-d₆):: δ= 10.1 (2s, 2H, exchangeable with D₂O, NH-Ph), 8.65 (broad signal, 2H, exchangeable with D₂O, 2H, CO-NH), 5.45 (m, 2H, CH), 5.21 (m, 2H, CH), 4.58 (m, 4H), 4.31 (m, 4H), 3.91 (m, 2H), 3.65-3.4 (m, 6H), 3.48 (s, 2H, COCH₂CO), 3.02 and 2.75 (2s, 6H, CONHCH₃), 2.13 (s, 12H, COCH₃), 2.06 (s, 12H, COCH₃).
- ¹³C-NMR (200 MHz, DMSO-d₆):: δ= 173.8 (CO), 172.1 (CO), 170.1 (CO), 168.0 (CO), 165. 1 (CO), 136.2, 136.0, 126.4, 121.9 (C-Br), 121.7 (C-Br), 121.6 (C-Br), 68.3 (CH), 66.7 (CH), 62.9 (CH₂), 62.4 (CH₂), 46.5 (CH₂), 45.9 (CH₂), 39.7 (CH₂), 33.5 and 28.1 (NHCH₃), 24.1 (CH₃), 20.5 (CH₃).

### b) N,N'-bis-[2,4,6-tribromo-3-[N-(2,3-diacetoxypropyl)-N-methylcarbamoyl]-5-[N-(2,3-diacetoxypropyl)carbamoyl]phenyl]-N,N'-dimethylmalonamide

A suspension of N,N'-bis-[2,4,6-tribromo-3-[N-(2,3-diacetoxypropyl)-N-methylcarbamoyl]-5-[N-(2,3-(diacetoxypropyl)carbamoyl]phenyl]malonamide (**example 22a**) (70 g, 0.045 moles) and anhydrous potassium carbonate (18.6 g , 0.135 moles) in acetone (490 mL) was cooled in an ice bath. Dimethyl sulfate (17.2 mL, 0.18 moles) was then added over fifteen-minute period. After which time the ice bath was removed and the reaction allowed to proceed overnight to room temperature.

The mineral salts were removed by filtration. The acetone was evaporated under reduce pressure and the residue dissolved in dichloromethane (400 mL) and water (400 ml) The dichloromethane layer was separated, washed with 5 % NaHCO₃ solution (3 x 200 mL), water (200 mL), dried over Na₂SO₄ and evaporated under pressure to give N,N'-bis-[2,4,6-tribromo-3-[N-(2,3-diacetoxypropyl)-N-methylcarbamoyl]-5-[N-(2,3-(diacetoxypropyl)carbamoyl]phenyl]-N,N'-dimethylmalonamide (62.7 g, 87.6 %). This product was used as such in the next step.
- ¹H-NMR (200 MHz, DMSO-d₆):: δ= 8.64 (broad signal, 2H, exchangeable with D₂O, 2H, CO-NH), 5.40 (m, 2H, CH), 5.24 (m, 2H, CH), 4.65 (m, 4H), 4.35 (m, 4H) 3.96 (m, 2H), 3.65 (m, 2H), 3.56 (m, 2H), 3.48 (s, 2H, COCH₂CO), 3.41 (m, 2H), 3.35 and 3.15 (2s, 6H, Ph-NCH₃), 3.02 and 2.75 (2s, 6H, CONHCH₃), 2.13 (s, 12H, COCH₃), 2.06 (s,12H, COCH₃).
- ¹³C-NMR (200 MHz, DMSO-d₆):: δ= 170.0 (CO), 170.35 (CO), 169.5 (CO), 165.0 (CO), 145.2, 139.6, 139.4, 129.7 (C-Br)), 129.6 (C-Br), 123.8 (C-Br), 68.3 (CH), 66.7 (CH), 62.8 (CH₂), 62.4 (CH₂), 46.5 (CH₂), 41.5 (CH₂), 38.6 and 36.3 (Ph-NHCH₃), 35.6 and 33.2 (COHN-CH₃), 24.1 (COCH₃), 20.5 (COCH₃).

### c) N,N'-bis-[2,4,6-tribromo-3-[N-(2,3-dihydroxypropyl)-N-methylcarbamoyl]-5-[N-(2,3-dihydroxypropyl)carbamoyl]phenyl]-N,N '-dimethylmalonamide

N,N'-bis-[2,4,6-tribromo-3-[N-(2,3-diacetoxypropyl)-N-methylcarbamoyl]-5-[N-(2,3-(diacetoxypropyl)carbamoyl]phenyl]-N,N'-dimethylmalonamide **(example 22b)** (55 g, 0.035 moles) was dissolved in 1:1 water/methanol (220 mL) and the pH adjusted to approximately 11 with 5 % sodium hydroxide. The solution was heated at 40 °C for two hours and then chilled and neutralised (pH 5-6) with concentrated hydrochloric acid.

The resulting solution was concentrated to dryness and the residue redissolved in methanol to remove mineral salts by filtration. The methanol was evaporated yielding crude N,N'-bis-[2,4,6-tribromo-3-[N-(2,3-dihydroxypropyl)-N-methylcarbamoyl]-5-[N-(2,3-(dihydroxypropyl)carbamoyl]phenyl]-N,N'-dimethylmalonamide (38 g).

The crude product was purified by reverse-phase preparative liquid chromatography. After evaporation and drying in vacuo, 34.6 g of N,N'-bis-[2,4,6-tribromo-3-[N-(2,3-dihydroxypropyl)-N-methylcarbamoyl]-5-[N-(2,3-(dihydroxypropyl)carbamoyl]phenyl]-N,N'-dimethylmalonamide were obtained in an overall (reaction-purification) yield of 79.0 %.
- ¹H-NMR (200 MHz, DMSO-d₆):: δ= 8.7 (broad signal, 2H, exchangeable with D₂O, 2H, CO-NH), 5.84 (m, 2H, OH), 5.72 (m, 2H, OH), 4.71 (m, 2H, OH), 4.68 (m, 2H, OH) 3.9-3.2 (m, 20H), 3.48 (s, 2H, COCH₂CO), 3.38 and 3.0 (2s, 6H, Ph-NCH₃), 3.08 and 2.62 (2s, 6H, CONHCH₃).
- ¹³C-NMR (200 MHz, DMSO-d₆):: δ= 169.5 (CO), 169.4 (CO), 166.3 (CO), 145.2, 139.4, 139.3, 128.9 (C-Br), 128.8 (C-Br), 122.3 (C-Br), 71.0 (CH), 69.4 (CH), 67.0 (CH₂), 66.5 (CH₂), 49.0 (CH₂), 41.9 (CH₂), 41.5 (CH₂), 37.7 and 35.9 (PhNCH₃), 34.9 and 31.2 (CONCH₃).

### Example 23

### Synthesis of N,N'-bis-[2,4,6-tribromo-3-[N-(2,3-dihydroxypropyl)-N-methylcarbamoyl]-5-[N-(2,3-dihydroxypropyl)carbamoyl]phenyl]-N,N'-bis-(2-hydroxyethyl)diglycolamide

### a) N,N'-bis-[2,4,6-tribromo-3-[N-(2,3-diacetoxypropyl)-N-methylcarbamoyl]-5-[N-(2,3-diacetoxypropyl)carbamoyl]phenyl]diglycolamide

Into a cooled (0-5°C) mixture of N,N'-bis-[2,4,6-tribromo-3-chlorocarbonyl-5-[N-(2,3-diacetoxypropyl)carbamoyl]phenyl]diglycolamide (**example 20a**) (80 g, 0.062 moles) and sodium carbonate decahydrate (53.02 g, 0.186 moles) in DMF (240 mL) was added dropwise a solution of 3-methylamino-1,2-propanediol (16.3 g, 0.155 moles) in DMF (40 mL) over 25-minute period. When the addition was complete, the reaction mixture was allowed to warm to room temperature and stirred for 24 hours.

The mineral salts were removed by filtration. The DMF was evaporated under reduced pressure and the residue dissolved in methanol (160 mL) and precipitated by adding into 2-propanol (1.5 L). The resulting precipitate was collected by filtration, washed and dried in vacuo.

The collected precipitate was dissolved in DMA (180 mL) and 4-dimethylaminopyridina (0.38 g, 0.0031moles) added. An ice bath was applied, and acetic anhydride (35.1 mL, 0.372moles) was added at such a rate as to maintain the temperature below 10°C. The ice bath was removed and the solution was stirred at 45°C for 16 hours.

After this period, water (600 mL) was added to the mixture reaction and stirred for 30 minutes. The mixture was extracted with dichloromethane (400 mL). The dichloromethane layer was separated, washed with 5 % NaHCO₃ solution (3 x 250 mL), water (250 mL), dried over Na₂SO₄ and evaporated under pressure to give N,N'-bis-[2,4,6-tribromo-3-[N-(2,3-diacetoxypropyl)-N-methylcarbamoyl]-5-[N-(2,3-(diacetoxypropyl)carbamoyl]phenyl]diglycolamide (95.2 g, 96.5 %). This product was used as such in the next step.
- ¹H-NMR (200 MHz, DMSO-d₆):: δ= 9.97 (2s, 2H, exchangeable with D₂O, NH-Ph), 8.70 (broad signal, 2H, exchangeable with D₂O, 2H, CO-NH), 5.41 (m, 2H, CH), 5.31 (m, 2H, CH), 4.57 (m, 4H), 4.37 (m, 4H), 3.99-3.4 (m, 8H), 3.94 (s, 4H, COCH₂OCH₂CO), 3.08 and 2.80 (2s, 6H, CONHCH₃), 2.21, 2.18, 2.10, 2.06 (4s, 24H, COCH₃).
- ¹³C-NMR (200 MHz, DMSO-d₆):: δ= 172.0 (CO), 170.35 (CO), 168.0 (CO) 166.5 (CO), 165.0 (CO), 136.2, 136.0, 126.0, 121.9 (C-Br)), 121.5 (C-Br), 126.0 (C-Br), 72.8 (CH₂), 68.3 (CH), 66.5 (CH), 62.9 (CH₂), 62.4 (CH₂), 46.6 (CH₂), 39.7 (CH₂), 32.1 and 29.7 (COHN-CH₃), 24.2, 23.8, 21.5, 20.4 (COCH₃).

### b) N,N'-bis-[2,4,6-tribromo-3-[N-(2,3-diacetoxypropyl)-N-methylcarbamoyl]-5-[N-(2,3-diacetoxypropyl)carbamoyl]phenyl]-N,N'-bis-(2-hydroxyethyl)diglycolamide

To a suspension of N,N'-bis-[2,4,6-tribromo-3-[N-(2,3-diacetoxypropyl)-N-methylcarbamoyl]-5-[N-(2,3-(diacetoxypropyl)carbamoyl]phenyl]dimalonamide (**example 23a**) (90g, 0.056moles) and anhydrous potassium carbonate (32.5 g , 0.23 moles) in methanol (360 mL) 2-chloroethanol (15 mL, 0.224 moles) was added all at once and the mixture was stirred and kept at 40°C for 4 hours and then left at room temperature overnight.

The mineral salts were removed by filtration. The methanol was evaporated under reduce pressure and to the resulting residue dichloromethane (400 mL) and water (400 mL) were added. The dichloromethane layer was separated, washed with 5 % NaHCO₃ solution (3 x 200 mL), water (200 mL), dried over Na₂SO₄ and evaporated under pressure to give N,N'-bis-[2,4,6-tribromo-3-[N-(2,3-diacetoxypropyl)-N-methylcarbamoyl]-5-[N-(2,3-diacetoxypropyl)carbamoyl]phenyl]-N,N'-bis-(2-hydroxyethyl)diglycolamide (81.2 g, 86.4 %). This product was used as such in the next step.
- ¹H-NMR (200 MHz, DMSO-d₆):: δ= 8.75 (broad signal, 2H, exchangeable with D₂O, 2H, CO-NH), 5.38 (m, 2H, CH), 5.14 (m, 2H, CH), 4.57 (m, 4H), 4.37 (m, 4H), 4.01 (s, 4H, COCH₂OCH₂CO), 3.99-3.4 (m, 16H), 3.08 and 2.80 (2s, 6H, CONHCH₃), 2.21, 2.18, 2.10, 2.06 (4s, 24H, COCH₃).
- ¹³C-NMR (200 MHz, DMSO-d₆):: δ= 172.0 (CO), 171.1 (CO), 168.8 (CO) 164.1 (CO), 163.1 (CO), 144.2, 139.0, 138.9, 128.4 (C-Br)), 128.3 (C-Br), 124.0 (C-Br), 71.8 (CH₂), 68.1 (CH), 66.7 (CH), 62.5 (CH₂), 62.3 (CH₂), 58.3 (CH₂), 51.4 (CH₂), 46.7 (CH₂), 39.3 (CH₂), 31.5 and 28.6 (COHN-CH₃), 24.1, 23.6, 21.3, 20.6 (COCH₃).

### c) N,N'-bis-[2,4,6-tribromo-3-[N-(2,3-dihydroxypropyl)-N-methylcarbamoyl]-5-[N-(2,3-dihydroxypropyl)carbamoyl]phenyl]-N,N'-bis-(2-hydroxyethyl)diglycolamide

N,N'-bis-[2,4,6-tribromo-3-[N-(2,3-diacetoxypropyl)-N-methylcarbamoyl]-5-[N-(2,3-diacetoxypropyl)carbamoyl]phenyl]-N,N'-bis-(2-hydroxyethyl)diglycolamide (**example 23b**) (80 g, 0.0476 moles) was dissolved in 1:1 water/methanol (320 mL) and the pH adjusted to approximately 11 with 5 % sodium hydroxide. The solution was heated at 40 °C for two hours and then chilled and neutralised (pH 5-6) with concentrated hydrochloric acid.

The resulting solution was concentrated to dryness, the residue redissolved in methanol and mineral salts removed by filtration. The methanol was evaporated yielding crude N,N'-bis-[2,4,6-tribromo-3-[N-(2,3-dihydroxypropyl)-N-methylcarbamoyl]-5-[N-(2,3-dihydroxypropyl)carbamoyl]phenyl]-N,N'-bis-(2-hydroxyethyl)diglycolamide (59.2 g).

The crude product was purified by reverse-phase preparative liquid chromatography. After evaporation and drying in vacuo, 51.3 g of N,N'-bis-[2,4,6-tribromo-3-[N-(2,3-dihydroxypropyl)-N-methylcarbamoyl]-5-[N-(2,3-dihydroxypropyl)carbamoyl]phenyl]-N,N'-bis-(2-hydroxyethyl)diglycolamide were obtained in an overall (reaction-purification) yield of 79.8 %.
- ¹H-NMR (200 MHz, DMSO-d₆):: δ= 8.7 (broad signal, 2H, exchangeable with D₂O, 2H, CO-NH), 5.84-4.68 (10H, exchangeable with D₂O, OH) 4.15 (s, 4H, COCH₂OCH₂CO), 3.89-3.22 (m, 28H), 3.18 and 2.71 (2s, 6H, CONHCH₃).
- ¹³C-NMR (200 MHz, DMSO-d₆):: δ= 168.8 (CO) 164.1 (CO), 163.1 (CO), 144.2, 139.0, 138.9, 128.4 (C-Br), 128.3 (C-Br), 124.0 (C-Br), 73.7 (CH₂), 71.0 (CH), 69.4 (CH), 67.0 (CH₂), 66.5 (CH₂), 58.2 (CH₂), 51.4 (CH₂), 49.01 (CH₂), 41.9 (CH₂), 31.9 and 26.3 (COHN-CH₃).

### Example 24

### Synthesis of N,N'-bis-[2,4,6-tribromo-3-[N-(2,3-dihydroxypropyl)-N-methylcarbamoyl]-5-[N-(2,3-dihydroxypropyl)carbamoyl]phenyl]-N,N'-bis-(2,3-dihydroxypropyl)diglycolamide

### a) N,N'-bis-[2,4,6-tribromo-3-[N-(2,3-diacetoxypropyl)-N-methylcarbamoyl]-5-[N-(2,3-diacetoxypropyl)carbamoyl]phenyl]-N,N'-bis-(2,3-dihydroxypropyl)diglycolamide

To a suspension of N,N'-bis-[2,4,6-tribromo-3-[N-(2,3-diacetoxypropyl)-N-methylcarbamoyl]-5-[N-(2,3-(diacetoxypropyl)carbamoyl]phenyl]diglycolamide (**example 23a**) (85 g, 0.0534 moles) and anhydrous potassium carbonate (30.9 g, 0.224 moles) in methanol (340 mL) 3-chloro-1,2-propanediol (23.5 g, 0.213 moles) was added all at once and the mixture was stirred and kept at 40°C for 4 hours and then left at room temperature overnight.

The mineral salts were removed by filtration. The methanol was evaporated under reduce pressure and the residue dissolved in dichloromethane (500 mL) and water (500 mL) The dichloromethane layer was separated, washed with 5 % NaHCO₃ solution (3 x 250 mL), water (250 mL), dried over Na₂SO₄ and evaporated under pressure to give N,N'-bis-[2,4,6-tribromo-3-[N-(2,3-diacetoxypropyl)-N-methylcarbamoyl]-5-[N-(2,3-diacetoxypropyl)carbamoyl]phenyl]-N,N'-bis-(2,3-dihydroxypropyl)diglycolamide (86.5 g, 93.2 %). This product was used as such in the next step.
- ¹H-NMR (200 MHz, DMSO-d₆):: δ= 8.55 (broad signal, 2H, exchangeable with D₂O, 2H, CO-NH), 5.8-4.7 (4m, 4H, exchangeable with D₂O, OH), 5.38 (m, 2H, CH), 5.14 (m, 2H, CH), 4.68-4,23 (m, 10H), 4.31 (s, 4H, COCH₂OCH₂CO), 3.90-3.4 (m, 16H), 3.08 and 2.80 (2s, 6H, CONHCH₃), 2.21, 2.18, 2.10, 2.06 (4s, 24H, COCH₃).
- ¹³C-NMR (200 MHz, DMSO-d₆):: δ= 172.0 (CO), 170.3 (CO), 168.0 (CO) 165.0 (CO), 162.9 (CO), 143.7, 139.3, 139.1, 129.5 (C-Br), 129.4 (C-Br), 123.7 (C-Br), 75.7 (CH₂), 70.8 (CH), 68.3 (CH), 66.8 (CH), 66.7 (CH₂), 62.8 (CH₂), 62.3 (CH₂), 49.9 (CH₂), 46.5 (CH₂), 39.6 (CH₂), 31.3 and 24.6 (COHN-CH₃), 23.9, 23.1, 21.1, 20.2 (COCH₃).

### b) N,N'-bis-[2,4,6-tribromo-3-[N-(2,3-dihydroxypropyl)-N-methylcarbamoyl]-5-[N-(2,3-dihydroxypropyl)carbamoyl]phenyl]-N,N'-bis-(2,3-dihydroxypropyl)diglycolamide

N,N'-bis-[2,4,6-tribromo-3-[N-(2,3-diacetoxypropyl)-N-methylcarbamoyl]-5-[N-(2,3-diacetoxypropyl)carbamoyl]phenyl]-N,N'-bis-(2,3-dihydroxypropyl)diglycolamide **(example 24a)** (90 g, 0..0517moles) was dissolved in 1:1 water/methanol (360 mL) and the pH adjusted to approximately 11 with 5 % sodium hydroxide. The solution was heated at 40 °C for two hours and then chilled and neutralized (pH 5-6) with concentrated hydrochloric acid.

The resulting solution was concentrated to dryness and the residue redissolved in methanol to remove mineral salts by filtration. The methanol was evaporated yielding crude N,N'-bis-[2,4,6-tribromo-3-[N-(2,3-dihydroxypropyl)-N-methylcarbamoyl]-5-[N-(2,3-dihydroxypropyl)carbamoyl]phenyl]-N,N'-bis-(2,3-dihydroxypropyl)diglycolamide (68.2 g).

The crude product was purified by reverse-phase preparative liquid chromatography. After evaporation and drying in vacuo, 61.2 g of N,N'-bis-[2,4,6-tribromo-3-[N-(2,3-dihydroxypropyl)-N-methylcarbamoyl]-5-[N-(2,3-dihydroxypropyl)carbamoyl]phenyl]-N,N'-bis-(2,3-dihydroxypropyl)diglycolamide were obtained in an overall (reaction-purification) yield of 84.4 %.
- ¹H-NMR (200 MHz, DMSO-d₆):: δ= 8.80 (broad signal, 2H, exchangeable with D₂O, CO-NH), 5.6-4.4 (12H, exchangeable with D₂O, OH) 4.31 (s, 4H, COCH₂OCH₂CO), 3.98-3.32 (m, 30H), 3.21 and 2.62 (2s, 6H, CONHCH₃).
- ¹³C-NMR (200 MHz, DMSO-d₆):: δ= 169.4 (CO) 166.3 (CO), 162.9 (CO), 143.7, 139.2, 139.0, 128.8 (C-Br), 128.7 (C-Br), 122.2 (C-Br), 75.7 (CH₂), 71.0 (CH), 70.8 (CH), 69.3 (CH), 67.3 (CH₂), 66.8 (CH₂), 66.5 (CH₂), 49.9 (CH₂), 49.0 (CH₂), 41.8 (CH₂), 31.9 and 26.3 (COHN-CH₃).

### Example 25

### Synthesis of N,N'-bis-[2,4,6-tribromo-5-carbamoyl-3-[N-(2,3-dihydroxypropyl)carbamoyl]phenyl]-N,N'-bis-(2,3-dihydroxypropyl)-malonamide

### a) N,N'-bis-(2,4,6-tribromo-5-carbamoyl-3-chlorocarbonylphenyl)malonamide

To a stirred and refluxed solution of 5-amino-2,4,6-tribromo-3-carbamoylbenzoyl chloride **(example 11d)** (43.5 g, 0.1 moles) in dioxane was added distilled malonyl chloride (7.75 g, 0.055 moles) over a thirty-minute period. When the addition is complete, the reaction mixture was left under reflux for two hours.

The reaction mixture was chilled and the resulting precipitate filtered off, washed with dioxane and dried to obtain N,N'-bis-(2,4,6-tribromo-5-carbamoyl-3-chlorocarbonylphenyl)malonamide (42.7 g, 91 %). The product was used in the next step without further purification.
- ¹H-NMR (200 MHz, DMSO-d₆):: δ= 10.3 (2s, 2H, exchangeable with D₂O, NH-Ph), 8.2 and 7.9 (2s, 4H, exchangeable with D₂O, CONH₂), 3.46 (s, 2H, COCH₂CO).
- ¹³C-NMR (200 MHz, DMSO-d₆):: δ= 173.7 (CO), 167.1 (CO), 166.7(CO), 141.2, 136.8, 131.9, 122.6 (C-Br), 122.3 (C-Br), 122.2 (C-Br), 45.9 (CH₂).

### b) N,N'-bis-[2,4,6-tribromo-5-carbamoyl-3-[N-(2,3-diacetoxypropyl)carbamoyl]phenyl]malonamide

Into a cooled (0-5°C) mixture of N,N'-bis-(2,4,6-tribromo-5-carbamoyl-3-chlorocarbonylphenyl)malonamide **(example 25a)** (50.7 g, 0.054 moles) and sodium carbonate decahydrate (46.3 g, 0.162 moles) in DMF (204 mL) was added dropwise a solution of 3-amino-1,2-propanediol (12.3 g, 0.135 moles) in DMF (37 mL) over 25-minute period. When the addition was complete, the reaction mixture was allowed to reach room temperature and stirred for 24 hours.

The mineral salts were removed by filtration. The DMF was evaporated under reduced pressure and the residue dissolved in methanol (136 mL) and precipitated by pouring into 2-propanol (1.1 L), The resulting precipitate was collected by filtration, washed and dried in vacuo.

The collected precipitate was dissolved in DMA (136 mL) and 4-dimethylaminopyridina (0.33 g, 0.027 moles) added. An ice bath was applied, and acetic anhydride (30.2 mL, 0.32 moles) was added at such a rate as to maintain the temperature below 10°C. The ice bath was removed and the solution was stirred at 45°C for 16 hours.

After this period, water (500 mL) was added to the mixture reaction and stirred for 30 minutes. The mixture was extracted with dichloromethane (300 mL). The dichloromethane layer was separated, washed with 5 % NaHCO₃ solution (3x200 mL), water (200 mL), dried over Na₂SO₄ and evaporated under pressure to give N,N'-bis-[2,4,6-tribromo-5-carbamoyl-3-[N-(2,3-diacetoxypropyl)carbamoyl]phenyl]malonamide (52.5 g, 80 %). This product was used as such in the next step.
- ¹H-NMR (200 MHz, DMSO-d₆):: δ= 9.8 (2s, 2H, exchangeable with D₂O, NH-Ph), 8.8 (broad signal, 2H, exchangeable with D₂O, CONH₂), 8.3 and 7.9 (2s, 4H, exchangeable with D₂O, CONH₂), 5.22 (m, 2H), 4.58 (m, 2H), 4.37 (m, 2H), 3.65-3.47 (m, 4H), 3.48 (s, 2H, COCH₂CO), 2.13 and 20.6 (2s, 12H, CH₃).
- ¹³C-NMR (200 MHz, DMSO-d₆):: δ= 173.7(CO), 172.0 (CO), 170.3 (CO), 167.1 (CO), 165.0 (CO), 136.7, 136.4, 127.1, 121.6 (C-Br), 121.3 (C-Br), 121.0 (C-Br), 68.3 (CH), 62.2 (CH₂), 45.9 (CH₂), 41.2 (CH₂), 24.1 (CH₃), 20.5 (CH₃).

### c) N,N'-bis-[2,4,6-tribromo-5-carbamoyl-3-[N-(2,3-dihydroxypropyl)carbamoyl]phenyl]-N,N'-bis-(2,3-dihydroxypropyl)malonamide

To a suspension of N,N'-bis-[2,4,6-tribromo-5-carbamoyl-3-[N-(2,3-diacetoxypropyl)carbamoyl]phenyl]malonamide **(example 25b)** (36.5 g, 0.03 moles) and anhydrous potassium carbonate (17 g, 0.123 moles) in methanol (340 mL) 3-chloro-1,2-propanediol (13.6 g, 0.123 moles) was added all at once and the mixture was stirred and kept at 40°C for 4 hours and then left at room temperature overnight.
The mineral salts were removed by filtration. The methanol was evaporated under reduce pressure and the residue dissolved in dichloromethane (500 mL) and water (500 mL) The dichloromethane layer was separated, washed with 5 % NaHCO₃ solution (3x250 mL), water (250 mL), dried over Na₂SO₄ and evaporated under pressure to give N,N'-bis-[2,4,6-tribromo-5-carbamoyl-3-[N-(2,3-diacetoxypropyl)carbamoyl]phenyl]-N,N'-bis-(2,3-dihydroxypropyl)malonamide (37.6 g).

This residue was dissolved methanol/water 1:1 (150 mL) and the pH adjusted to approximately 11 with 5% NaOH. The solution was heated at 50°C for 2 h and then chilled and neutralized (pH=6.5) with hydrochloric acid 1:1. The resulting solution was evaporated to dryness and the residue redissolved in methanol to remove mineral salts by filtration. The methanol was evaporated to dryness under reduced pressure. The residue was purified by PLC (eluent: 5% methanol) to obtain N,N'-bis-[2,4,6-tribromo-5-carbamoyl-3-[N-(2,3-dihydroxypropyl)carbamoyl]phenyl]-N,N'-bis-(2,3-dihydroxypropyl)malonamide (25.1 g, 70%).
- ¹H-NMR (200 MHz, DMSO-d₆):: δ= 8.7 (broad signal, 2H, exchangeable with D₂O, CONH₂), 8.2 and 7.8 (2s, 4H, exchangeable with D₂O, CONH₂), 5.68-4.82 (4m, 8H, OH), 4.92 (m, 2H), 3.97-3.2 (m, 16H), 3.47 (s, 2H, COCH₂CO).
- ¹³C-NMR (200 MHz, DMSO-d₆):: δ= 169.2 (CO), 167.2 (CO), 166.3 (CO), 144.8, 139.7, 139.6, 128.9 (C-Br), 128.8 (C-Br), 122.4 (C-Br), 71.4 (CH), 70.8 (CH), 66.8 (CH₂), 66.5 (CH₂), 48.8 (CH₂), 42.5 (CH₂), 41.9 (CH₂).

### Example 26

### Synthesis of N,N'-bis-[2,4,6-tribromo-3,5-bis-[N-(1-hydroxymethyl-2,3-dihydroxypropyl)carbamoyl]phenyl]-N,N'-dimethylmalonamide

### a) N,N'-bis-(2,4,6-tribromo-3,5-dichlorocarbonylphenyl)malonamide

To a stirred and refluxed solution of 5-amino-2,4,6-tribromoisophthaloyl dichloride **(example 5b)** (45.5 g, 0.1 moles) in dioxane was added distilled malonyl chloride (7.75 g, 0.055 moles) over a thirty-minute period. When the addition is complete, the reaction mixture was left under reflux for two hours.

The reaction mixture was chilled and the resulting precipitate filtered off, washed with dioxane and dried to obtain N,N'-bis-(2,4,6-tribromo-3,5-dichlorocarbonylphenyl)malonamide (43.5 g, 89 %). The product was used in the next step without further purification.
- ¹H-NMR (200 MHz, DMSO-d₆):: δ = 3.45 (s, 2H, CO-CH₂-CO), 10.2 (m, 2H, exchangeable with D₂O, NH-Ph).
- ¹³C-NMR (200 MHz, DMSO-d₆):: δ = 45.62 (CH₂), 121.28/123.19/127.14 (C-Br), 137.32(aromatic C-N), 141.24 (aromatic C-COCl), 166.71 (CO in COCI group), 173.74 (CO in CONH group).

### b) N,N'-bis-[2,4,6-tribromo-3,5-bis-[N-(7-hydroxy-4,4-dimethyl-3,5-dioxepinyl)carbamoyl]phenyl]malonamide

To a solution of N,N'-bis-(2,4,6-tribromo-3,5-dichlorocarbonylphenyl)-malonamide (27.4 g, 0.028 moles) **(example 26a)** in DMF (265 mL), Na₂CO₃.10H₂O (35.2 g, 0.123 moles) and 7-amino-5-hydroxy-2,2-dimethyl-1,3-dioxepine (19.8 g, 0.123 moles) were added. The mixture was heated at reflux for four hours. The reaction medium was cooled at room temperature and filtered. The filtrate was evaporated to dryness under reduced pressure to obtain N,N'-bis-[2,4,6-tribromo-3,5-bis-[N-(7-hydroxy-4,4-dimethyl-3,5-dioxepinyl)carbamoyl]phenyl]malonamide (37.2 g , 90%). This product was used in next step without purification.
- ¹H-NMR (200 MHz, DMSO-d₆):: δ = 1.21 (s, 24H, 8CH₃), 3.48 (s, 2H, CO-CH₂-CO), 3.25-4.25 (m, 24H, 8CH₂, 8CH), 4.83 (d, 4H, exchangeable with D₂O, 4OH), 8.64 (bs, 4H, exchangeable with D₂O, 4CONH), 9.91 (s, 2H, exchangeable with D₂O, NH-Ph).
- ¹³C-NMR (200 MHz, DMSO-d₆):: δ = 24.20/24.49 (CH₃), 45.55 (CO-CH₂-CO), 53.21 (CH-N), 66.17/66.52 (CH₂-O), 70.19 (CH-O), 100.92 (C), 119.4/120.3 (C-Br), 127.22/135.32 (aromatic C), 166.34 (Ph-CONH), 173.45 (CONH-Ph).

### c) N,N'-bis-[2,4,6-tribromo-3,5-bis-[N-(1-hydroxymethyl-2,3-dihydroxypropyl)carbamoyl]phenyl]-N,N'-dimethylmalonamide

A suspension of N,N'-bis-[2,4,6-tribromo-3,5-bis-[N-(7-hydroxy-4,4-dimethyl-3,5-dioxepinyl)carbamoyl]phenyl]malonamide (37 g, 0.025 moles) (**example 26b**) and anhydrous potassium carbonate (10.37 g, 0.075 moles) in acetone (490 mL) was cooled in an ice bath. Dimethyl sulfate (9.5 mL, 0.1 moles) was then added over fifteen-minute period. After which time the ice bath was removed and the reaction allowed to proceed overnight to room temperature.

The mineral salts were removed by filtration. The acetone was evaporated under reduce pressure and the residue dissolved in dichloromethane (400 mL) and water (400 ml) The dichloromethane layer was separated, washed with 5 % NaHCO₃ solution (3x200 mL), water (200 mL), dried over Na₂SO₄ and evaporated under reduced pressure to give a crude of N,N'-bis-[2,4,6-tribromo-3,5-bis-[N-(7-hydroxy-4,4-dimethyl-3,5-dioxepinyl)carbamoyl]phenyl]-N,N'-dimethylmalonamide (35 g).

This residue was dissolved in methanol/water 1:1 (200 mL) and the pH adjusted to approximately 11 with 5 % sodium hydroxide. The solution was heated at 50 °C for two hours and then the reaction medium was acidulated (pH=3) with hydrochloric acid 1:1 and stirred at room temperature for an hour. Then, the reaction medium was neutralized with 5% NaOH. The solvents were evaporated to dryness under reduced pressure. The residue was purified by PLC (eluent 5% methanol) to obtain N,N'-bis-[2,4,6-tribromo-3,5-bis-[N-(1-hydroxymethyl-2,3-dihydroxypropyl)carbamoyl]phenyl]-N,N'-dimethylmalonamide (23.2 g, 69%).
- ¹H-NMR (200 MHz, DMSO-d₆):: δ=3.01/3.41 (s/s, 6H, 2CH₃-N), 3.48 (s, CO-CH₂-CO), 3.25-3.85 (m, 24H, 8CH₂, 8CH), 4.52-5.83 (m/m/m, 12H, 12OH), 8.76 (bs, 4H, exchangeable with D₂O, CONH).
- ¹³C-NMR (200 MHz, DMSO-d₆):: δ= 35.8/37.7 (CH₃-N), 41.5 (CO-CH₂-CO), 50.77 (CH-N), 60.84/64.39 (CH₂OH), 70.52 (CH-O), 121.35/128.32 (C-Br), 138.73/145.92 (aromatic C), 166.47 (Ph-CONH), 169.51 (CONH-Ph).

### Example 27

### a) Synthesis of 2,4,6-tribromobenzene-1,3,5-tricarboxylic acid-tris-(2,3-dihydroxypropyl-N-methyl)triamide

50 g tribromo-trimesic acid trichloride (EP 0 073 715, page 43) are dissolved in 100 mL dimethylacetamide at 50° C. Then a solution of 60g of N-methylamino-2,3-propanediol in 120 mL dimethylacetamide is added drowise within 20 minutes so that an internal temperature of 60° C is not exceeded. Subsequently, the mixture is stirred for 4 hours at 50° C after standing overnight hydrochloric acid is added to neutralize the solution. After concentration under vacuum at 50° C, the residue is dissolved in 100 mL of water and treated first with 1000 mL of a cation exchange resin and then the filtrate is treated with 1000 mL of a anion exchange resin. The colorless salt-free filtrate is then concentrated under vacuum to dryness and the residue is dried at 50° C to obtain a white powder (56 g, 80%); mp=148-150° C, Br: 33.85%.
- ¹H-NMR (200 MHz, DMSO-d₆):: δ = 2.86/3.08 (s,s, 9H, CH₂ and CH groups), 4.60/4.80 (m,m, 6H, 60H).
- ¹³C-NMR (200 MHz, DMSO-d₆):: δ = 33.95 (CH₃), 49.23 (CH₂-N), 61.81 (CH₂-O), 70.18 (CH), 117.33/121.85/122.94 (C-Br), 135.28/140.13/140.91 (aromatic C), 166.13 (CO)

Correspondingly there are obtained

### b) 2,4,6-tribromobenzene-1,3,5-tricarboxylicacid-tris-(1,3-dihydroxypropyl)triamide

Br: 35.99%
- ¹H-NMR (200 MHz, DMSO-d₆):: δ = 3.51/3.71 (m,m, 12H, 6CH₂), 3.98 (m, 3H, 3CH), 4.65 (m, 6H, 6OH).
- ¹³C-NMR (200 MHz, DMSO-d₆):: δ = 52.36 (CH), 63.56 (CH₂), 128.56 (C-Br), 131.57 (aromatic C), 167.14 (CO).

### c) 2,4,6-tribromobenzene-1,3,5-tricarboxylicacid-tris-(2-hydroxyethyl)triamide

Br: 41.61%
- ¹H-NMR (200 MHz, DMSO-d₆):: δ = 3.63 (m, 6H, 3CH₂-N), 3.79 (m, 6H, 3CH₂-O), 4.46 (m, 3H, 3OH).
- ¹³C-NMR (200 MHz, DMSO-d₆):: δ = 42.95 (CH₂-N), 61.62 (CH₂-O), 129.54 (C-Br), 132.28 (aromatic C), 167.17 (CO).

### d) 2,4,6-tribromobenzene-1,3,5-tricarboxylic acid-tris-(2-hydroxyethyl-N-methyl)triamide

Br: 38.78%
- ¹H-NMR (200 MHz, DMSO-d₆):: δ = 3.00 (s, 9H, 3CH₃), 3.44 (m, 6H, 3CH₂-N), 3.75 (m, 6H, 3CH₂-O), 4.28 (m, 3H, 3OH).
- ¹³C-NMR (200 MHz, DMSO-d₆):: δ = 34.23 (CH₃), 51.40 (CH₂-N), 57.68 (CH₂-O), 125.43 (C-Br), 131.44 (aromatic C), 169.47 (CO).

### Example 27a

### Phantom studies with conventional mammography

Iodine and bromine containing contrast media solutions **5-[N-(2,3-dihydroxypropyl)acetylamino]-2,4,6-triiodo-N,N'-bis-(2,3-dihydroxypropyl)isoph-thalamide** (Iohexol) and **example 4,** with concentrations of 10.51, 5.254, 2.627 and 1.313 mmol/L equivalent in moles to 4, 2,1 and 0.5 mg I/mL **Iohexol** were prepared in 1% agar. The agar pieces were prepared in cell-culture well-plates having a diameter of 24 mm and a final height of 10 mm. After formation of solid agar the agar discs were placed in a water phantom containing 5 cm height of water. Additionally a control agar disc prepared with physiological saline instead of CM volume was added. The whole phantom was imaged with a commercial available mammograph at various energies of 30 kV, 25 and 20 kV and 140 mAs. After exposure the mammographic films were developed according to standard quality guidelines. Later the films were digitized and signal intensities of the agar discs and surrounding water background were measured with a imaging analyzing software.

### Results

Iodine agar pieces could be detected down to concentration of 1 mg I/mL whereas bromine containing **example 4** showed higher absorption over the whole concentration range and was detectable down to concentrations of 1.31 mmol/L moles equivalent to 0.5 mg I/mL of **Iohexol.**

At equimolar concentrations **example 4** was about 50% more effective than iodine carrying **Iohexol.** This relation is also observed at energies of 25 and 20 kV indicating superior absorption characteristics of bromine over iodine in the entire energy range covered by mammography. Figure 1 shows a mammographic image at 30 kV and 140 mAs of a water phantom with contrast media containing agar discs. Left line 10 mm agar slices of **Iohexol.** Right line with 10 mm agar slices spiked with **example 4** Both contrast media have equimolar concentrations reaching from the top to the bottom from 10.51, 5.25, 2.63 and 1.313 mmol/L, equivalent to molar concentrations of 4, 2, 1, and 0.5 mg I/mL of **Iohexol.** Control agar slices with NaCl were not visible.

Figure 2 shows X-ray absorption of iodine and bromine containing contrast media **Iohexol** and **example 4** at 30, 25 and 20 kV energies commonly used in mammography, showing an absorption of 50 % higher of bromine at equimolar concentrations when compared to iodine at the entire range of energies used by mammography.

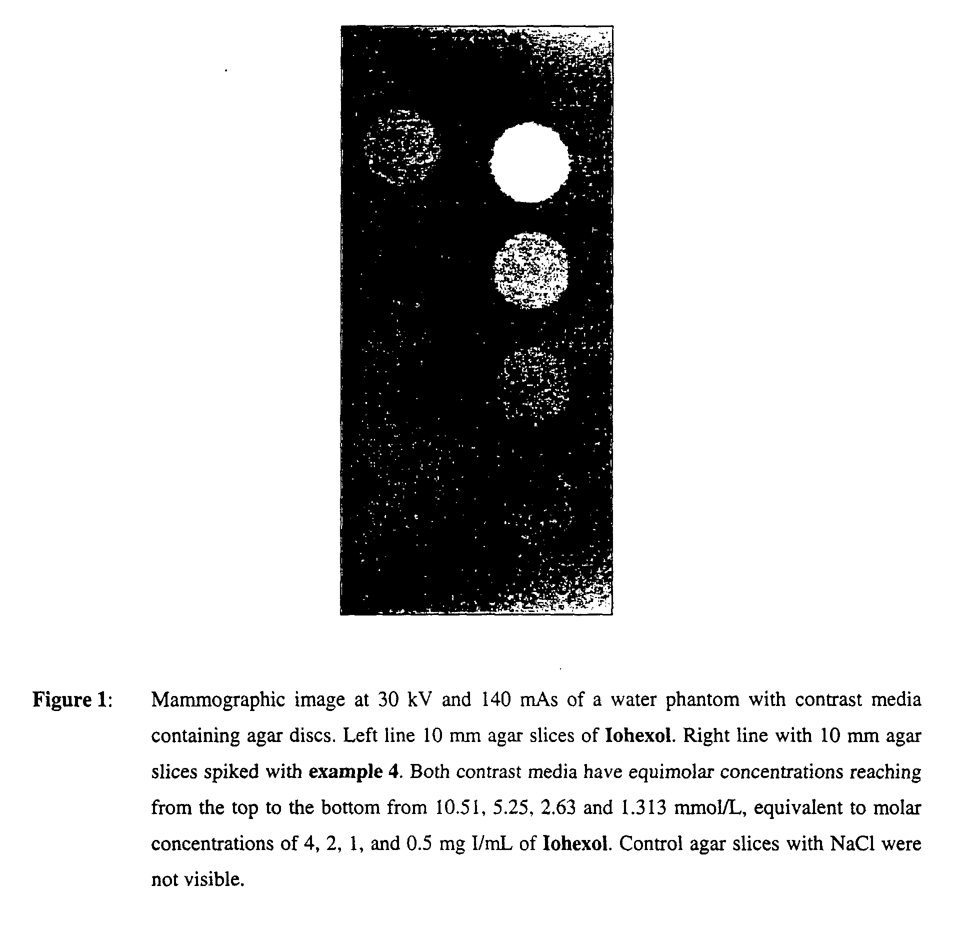

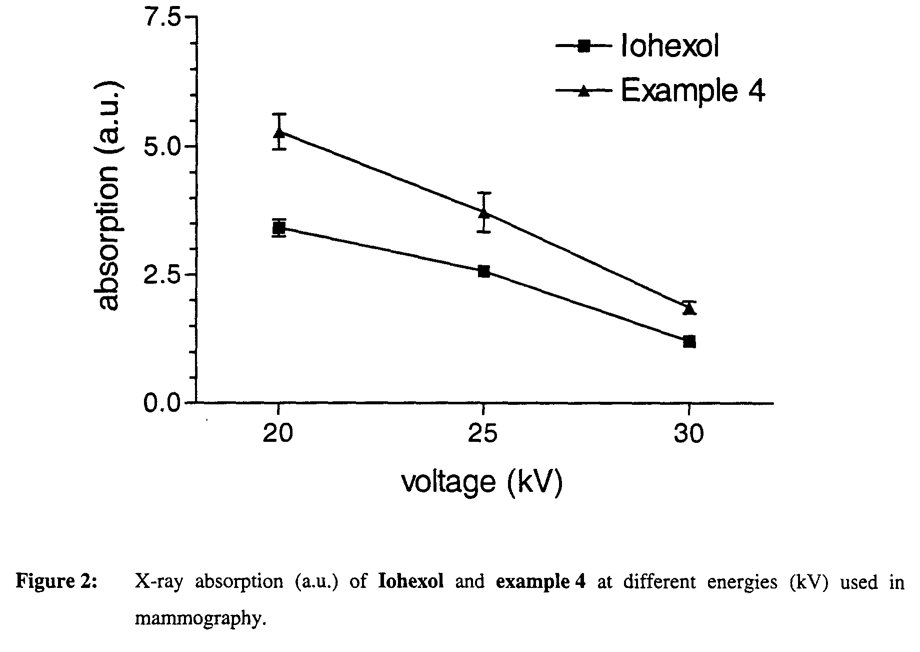

### Example 28

Physicochemical properties of new bromine containing contrast media **example 4, example 14, example 25 and example 26** were determined and compared to the corresponding iodinated compounds **Iohexol** and **N,N'-bis-[2,4,6-triiodo-3,5-bis-[N-(1-hydroxymethyl-2,3-dihydroxypropyl)carbamoyl]phenyl]-N,N'-dimethylmalonamide** (Iotrolan) and to the brominated compound **2,4,6-tribromo-5-[N-(2,3-dihydroxypropyl)acetylamino]-N,N'-bis-(2,3-dyhydroxypropyl)isophthalamide,.** For this purpose contrast media formulation with a molar concentration of 0.788 mmol/L for monomeric compounds and of 0.394 mmol/L for dimeric compounds, equivalent to a iodine concentration of 300 mgI/mL or 189 mgBr/mL were prepared at neutral pH. After heat sterilization under standard conditions for 20 min at 120°C changes in pH and free bromine and iodine was measured. The osmolality was measured at 37°C using a Knauer vapor pressure osmometer. Viscosity of contrast media formulations was determined using a Schott capillary viscosimeter which was standardized with water.

All bromine containing contrast media were stable during heat sterilization and showed no increase in free bromine and deviations from pH according to the guidelines and stability data were comparable to the iodinated reference compounds. The osmolality of brominated compounds is slightly higher, as a physicochemical consequence of the smaller atom size of bromine, the viscosity is slightly lower than the iodinated reference substance showing good physicochemical properties useful for the in-vivo administration, and higher molar percentage of bromine (table 3).

### Results

**Table 3:**

| Physicochemical data of CM containing iodine vs. contrast media with bromine. | | | |
|---|---|---|---|
| | **Osmolality** a) monomer, 0.788 mol/L, 37°C b) dimer, 0.394 mol/L, 37° C **mOsm/kg water** | **Viscosity (37°C) mPa·s** | **Aqueous solubility mgBr/mL** |
| **Iohexol**^{**a**} | 690 | 6.1 | |
| **2,4,6-Tribromo-5-[N-(2,3- dihydroxypropyl)acetylamino]-N,N'-bis- (2,3-dyhydroxypropyl)isophthalamide**^{**a**} | 908 | 4.6 | > 300 |
| Prior art: patent **EP 0 118 348 A1** (Guerbet) | | | |
| **Example 4**^{a} | 818 | 3.5 | >400 |
| **Example 14**^{a} | 500 | 3.4 | >400 |
| **Iotrolan**^{b} | 320 | 8.5 | >400 |
| **Example 25**^{b} | 320 | 5.5 | 400 |
| **Example 26**^{b} | 370 | 6.3 | 400 |

### Example 29

Influence of bromine containing contrast media **example 4, example 14, example 25 and example 26** on erythrocyte morphology was compared to the iodinated compounds **Iohexol, Iopromide** and **Iotrolan** in rat blood. Six parts by volume was mixed with one part by volume of each test substance. The mixtures were examined with and optical microscope immediately, 1 hour and 2 hours after incubation in terms of a damage index of blood cells.

### Results

The bromine contrast media **example 4, example 14, example 25 and example 26** showed substantially less influence on erythrocyte morphology than the iodinated reference substances. The damage index for the new synthesized brominated compounds two hours after incubation was improved for the new compounds compared to the iodinated CM (Table 4).

**Table 4:**

| Influence of **example 4, example 14, example 25 and example 26** on erythrocyte morphology after 2 h incubation period. Iodinated compounds **Iohexol and Iotrolan** served as references. | | | | | | | |
|---|---|---|---|---|---|---|---|
| | **Iohexol** | **Ex. 4** | **Ex. 14** | **Iotrolan** | **Ex. 25** | **Ex. 26** | **Saline** |
| Erythrocyte | 1.31 | 0.82 | 0.88 | 0.69 | 0.45 | 0.51 | 0.30 |
| morphology | ± 0.18 | ± 0.14 | ± 0.16 | ± 0.18 | ± 0.11 | ± 0.13 | ± 0.12 |
| Damage index 2 h | | | | | | | |

### Example 30

In order to estimate the acute tolerance of tribromobenzene contrast media the approximate LD₅₀ of **example 4, example 14, example 25 and example 26** was tested after single intravenous injection in mice. Iodinated compounds **Iohexol** and **Iotrolan** as well as the bromine compound **2,4,6-tribromo-5-[N-(2,3-dihydroxypropyl)acetylamino]-N,N'-bis-(2,3-dyhydroxypropyl)isophthalamide** and served as references. The contrast media were injected at a concentration of 189 mg Br/mL or 300 mg I/mL respectively, which are equimolar concentrations of 0.788 mol/L for monomeric, and 0.394 mol/L for dimeric contrast media into a tail vein of male Sprague-Dawley mice weighing 18-22 g. The behavior of the animals and the lethality was monitored for 7 days post injection.

### Results

Intravenous injections proved **2,4,6-tribromo-5-[N-(2,3-dihydroxypropyl)acetylamino]-N,N'-bis-(2,3-dihydroxypropyl)isophthalamide** and with an LD₅₀ approx. of 59 mmol/kg, to be well tolerated in rats with similar results found for Iohexol. Iotrolan showed with 63 mmol/kg an expected increases in tolerability compared to monomeric iodinated contrast media. In the case for the new synthesized brominated contrast media **example 4, example 14, example 25 and example 26** surprisingly an notable increase of the tolerability in the range of 13 - 23 % with respect to the iodinated and brominated reference compounds was observed (Table 5).

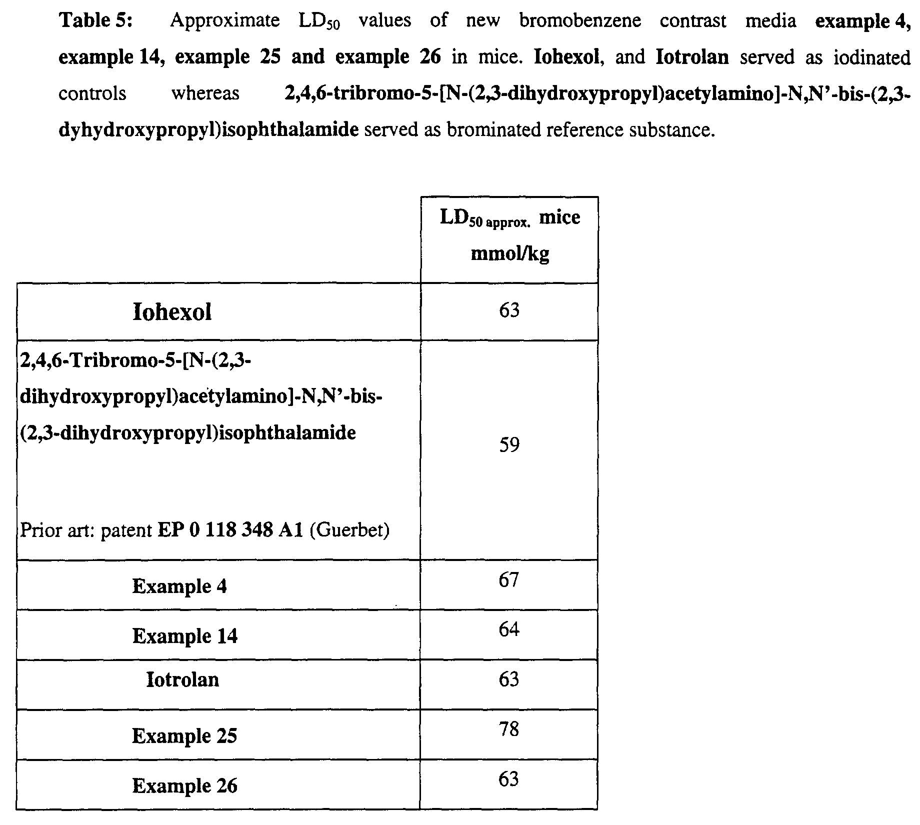

The tolerability of new synthesized bromine compounds **example 4, example 14, example 25** and **example 26** proved to be surprisingly higher than iodinated and brominated reference compounds

### Example 31

The pharmacokinetic profile of **example 4** was examined after intravenous administration of 3H-labelled **example 4** to male Sprague-Dawley rats. For this purpose **example 4** was labeled with tritium of a chemical concentration of 300 mg / mL and a radioactive concentration of 2.75 MBq/mL. After intravenous injection in the right jugular vein via a catheter at a dose of 600 mg EXAMPLE 4/kg blood samples of 400 µl were collected in plastic tubes at 0.083, 0.25, 0.5, 1, 2, 4, 6, 8 and 24 hours after administration. Plasma samples were obtained after blood sample centrifugation at 250 g for 10 min. After injection, the rats were transferred in metabolic cages allowing the collection of urine and faeces at different intervals. Aliquots of 100 µl of samples with blood or urine were added to 10 ml of scintillation cocktail and the radioactivity was measured by means of a Tricor-2500 TR automatic scintillation spectrometer. Faeces samples were freeze-dried for two days. They were milled and aliquots of 50 or 100 mg were taken, moistened with 1 mL water and digested with Soluene-350® and 1 mL of isopropyl alcohol at 50 °C for 24 hours. Digested faeces samples were bleached and radioactivity was measured as mentioned above. Plasma levels of **example 4** and mean cumulative urinary and faecal excretion data were fitted to a two-compartment model. Curve fit and evaluation of kinetic parameters was performed on the TOP-Fit v 2.0β program.

### Results

Plasma levels of **example 4** are shown in figure 3. Analysis of the data showed a good fit to a bicompartment model with a t_{½ α} of 11.4 min and a t_{½ β} = 31.2 min and a distribution volume V_{c} of = 0.26 L/kg. The elimination characteristics are summarized in figure 4 showing a rapid cumulative urinary excretion reaching 62.8% of injected dose at 24 h p.i. and a cumulative faecal excretion reaching 5.39% of injected dose at 24 h.

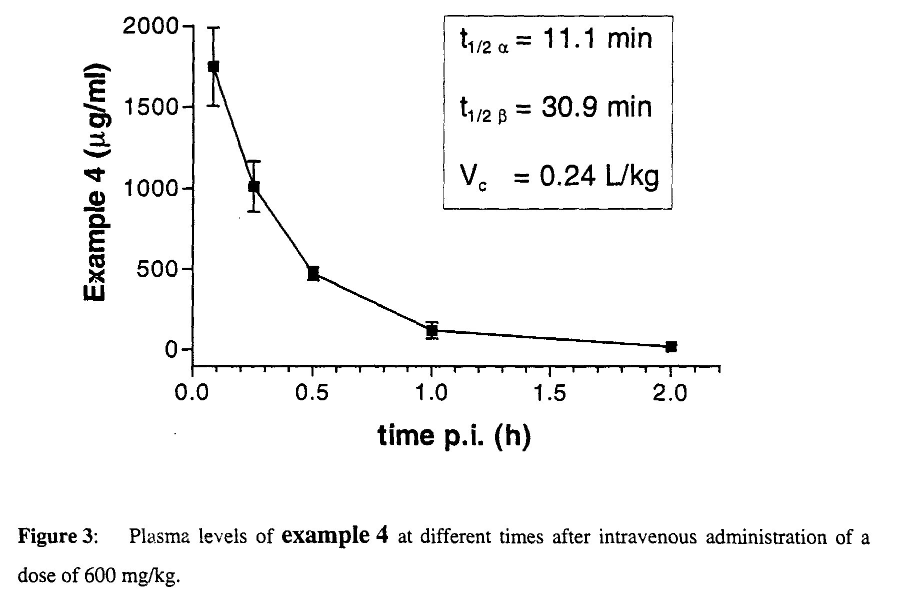

Analysis of bile fluid collected via a catheter in the bile duct and HPLC-analysis showed no metabolisation of **example 4** by the animal. The compound **example 4** was eliminated via the renal pathway with 63.7 % I.D. in the urine detected at 24 h p.i.. In faeces only minor amounts of 5.1 % I.D. were measured 24 h p.i. In summary **example 4** exhibits a pharmacokinetic profile of a hydrophilic low molecular weight non-ionic radiographic contrast agent.

### Radiological compositions.

In further accordance with the present invention, radiological composition may be prepared containing one of the aforementioned compounds of the invention as an X-ray contrast agent together with a pharmaceutically acceptable radiological vehicle.

Pharmaceutically acceptable vehicles include those that are suitable for injection such as aqueous buffer solutions; e.g. tris-(hydroxymethyl)aminomethane and its salts , phosphate, citrate, bicarbonate, etc., sterile water for injection, physiological saline and balanced ionic solutions containing chloride and/or bicarbonate salts of normal blood plasma cations such as Ca, Na, K and Mg.

The vehicles may contain a chelating amount, e.g., a small amount of ethylenediamine tetraacetic acid, the calcium disodium salt, or other pharmaceutically acceptable chelating agent.

The concentration of the contrast agent varies with the field of use. For mammography purposes the concentration of bromine is generally 10-400 mg/mL and the dose 20 to 500 mL.

As a non-limitative example below is given an example of a preparation of a

### Radiological composition

2,4,6-Tribromo-5-hydroxyacetylamino-N,N'-bis-(2,3-dihydroxypropyl)-N-methylisophthalamide, **example 4,** of the present invention, trometamol (TRIS) and the edetate (CaNa₂EDTA) are dissolved in water for injection (approximately 900 ml). The pH is adjusted to 7.3 with diluted HCl and water for injection is added to make the volume of solution up to 1000 mL.

The solution is then membrane filtered and dispensed in bottles or injection vials. The dispensed products are autoclaved for 20 minutes at 120 °C.

### Radiological composition

600 g 2.4.6-Tribromobenzene-1.3.5-tricarboxylic acid-tris (bis-2-hydroxyethyl)triamide (example 27 e of the present invention), and 100 mg Calcium disodium edetate are dissolved in water for injection (approximately 900 ml). The solution is neutralized (pH 7) by adding 1,23 g sodium bicarbonate. The volume is replenished to 1000 ml by addition of water for injection, and the thus obtained solution is then heat-sterilized.

## Claims

1. A compound of the formula I: 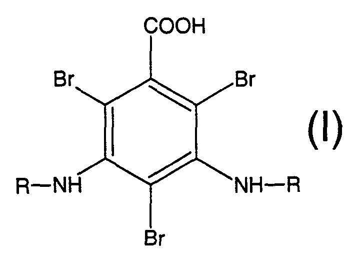 Wherein:
R is -COCH₂OH, -COCH₂OCH₃, -SO₂CH₂Br or -CO CH₃
as contrast media for the use in X-ray mammography.

2. A compound of the formula II: 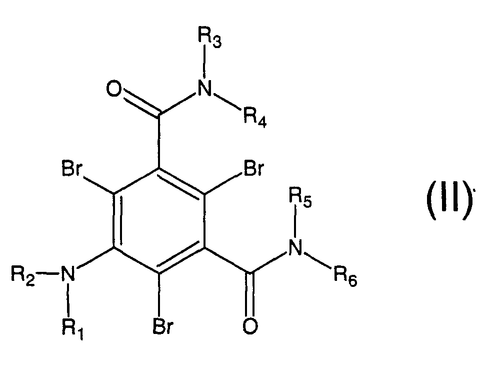 Wherein the substituents R₁, R₂, R₃, R₄, R₅, and R₆ are given in table 1 below,
as contrast media for the use in X-ray mammography,
**Table 1.**
| Compounds of formula **II** | | | | | |
|---|---|---|---|---|---|
| **R**_{**1**} | **R**_{**2**} | **R**_{**3**} | **R**_{**4**} | **R**_{**5**} | **R**_{**6**} |
| H | COCH₂OH | CH₃ | CH₂CHOHCH₂OH | H | CH₂CHOHCH₂OH |
| H | COCH₂OH | H | CH₂CHOHCH₂OH | H | CH₂CHOHCH₂OH |
| H | COCH₂OH | H | CH₂CHOHCH₂OH | H | CH₂CH₂OH |
| CH₃ | COCH₂OH | H | CH₂CHOHCH₂OH | H | CH₂CHOHCH₂OH |
| CH₂CH₂OH | COCH₂OH | H | CH₂CHOHCH₂OH | H | CH₂CHOHCH₂OH |
| CH₂CHOHCH₂OH | COCH₂OH | H | CH₂CHOHCH₂OH | H | CH₂CHOHCH₂OH |
| H | SO₂CH₂Br | CH₃ | CH₂CHOHCH₂OH | H | CH₂CHOHCH₂OH |
| H | COCH₂OH | CH₃ | CH₂CHOHCH₂OH | H | H |
| H | COCH₂OH | H | CH(CH₂OH)CHOHCH₂OH | H | H |
| H | COCH₂OH | CH₂CH₂OH | CH₂CH₂OH | CH₂CH₂OH | CH₂CH₂OH |
| CH₂CH₂OH | COCH₂OH | H | CH(CH₂OH)CHOHCH₂OH | H | H |
| H | COCH₂OH | CH₃ | CH₂CHOHCH₂OH | CH₃ | CH₂CHOHCH₂OH |
| H | COCH₂OCH₃ | CH₃ | CH₂CHOHCH₂OH | CH₃ | CH₂CHOHCH₂OH |
| H | COCH₂OH | H | CH₂CHOHCH₂OH | H | CH(CH₂OH)₂ |
| CH₂CH₂OH | COCH₃ | H | CH(CH₂OH)₂ | H | H |
| CH₂CH₂OH | COCH₃ | H | CH(CH₂OH)CHOHCH₂OH | H | H |

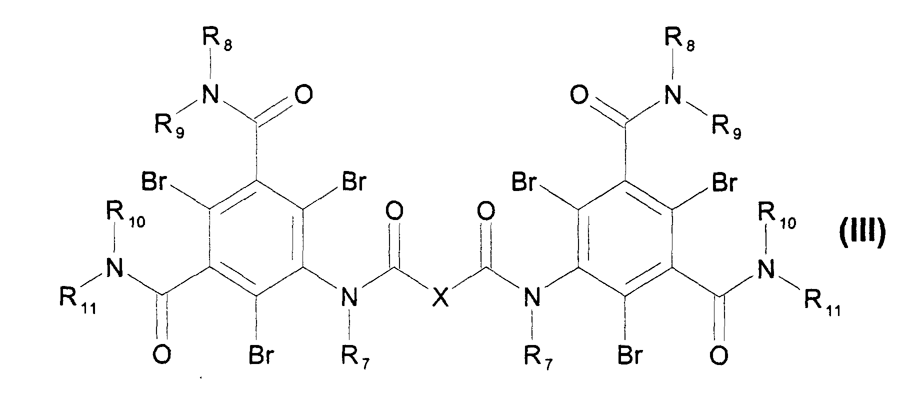

3. wherein
R₇ is hydrogen, straight-chain or branched C₁-C₄-alkyl or mono-or polyhydroxyalkyl,
R₈ is hydrogen, straight-chain or branched C₁-C₄-alkyl or mono-or polyhydroxyalkyl,
R₉ is mono-or polyhydroxyalkyl,
R₁₀ is hydrogen, straight-chain or branched C₁-C₄-alkyl or mono-or polyhydroxyalkyl,
R₁ ₁ is hydrogen, straight-chain or branched C₁-C₄-alkyl or mono-or polyhydroxyalkyl,
X is straight-chain or branched C₁-C₄-alkyl or C₁-C₄-alkoxy-C₁-C₄-alkyl,
with the proviso, that the compound 5.51-[N,N'-(hydroxy-2-ethyl) malonyldiimino] bis [2.4.6-tribromo-N,N'-bis-(2-methyl-1.3-dihydroxy-2-propyl)] isophthalamide is excluded,
as contrast media for the X-ray mammography.

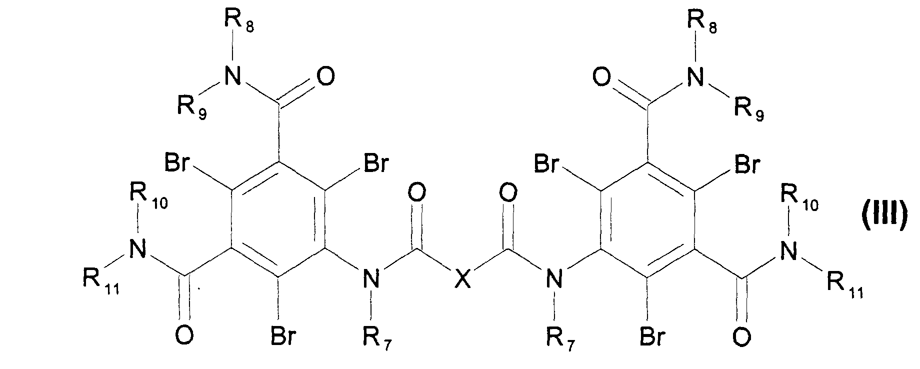

4. wherein
R₇ is hydrogen, straight-chain or branched C₁-C₄-alkyl or mono-or polyhydroxyalkyl,
R₈ is hydrogen, straight-chain or branched C₁-C₄-alkyl or mono-or polyhydroxyalkyl,
R₉ is mono-or polyhydroxyalkyl,
R₁₀ is hydrogen, straight-chain or branched C₁-C₄-alkyl or mono-or polyhydroxyalkyl,
R₁₁ is hydrogen, straight-chain or branched C₁-C₄-alkyl or mono-or polyhydroxyalkyl,
X is straight-chain or branched C₁-C₄-alkyl or C₁-C₄-alkoxy-C₁-C₄-alkyl,
with the proviso, that the compound 5,5'-[N,N'-(hydroxy-ethyl) malonyldiimino] bis [2.4.6-tribromo-N,N'-bis-(2-methyl-1.3-dihydroxy-2-propyl)] isophthalamide is excluded.

5. A compound of claim 1 which is 2,4,6-tribromo-3,5-dihydroxyacetylaminobenzoic acid, example 1. 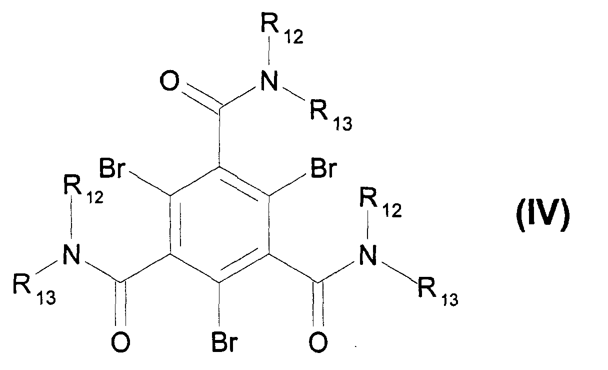 wherein
R₁₂ is hydrogen, straight-chain or branched C₁-C₄-alkyl or mono-or polyhydroxyalkyl-C₁-C₄-alkyl,
R₁₃ is straight-chain or branched C₁-C₄-alkyl or mono-or polyhydroxyalkyl-C₁-C₄-alkyl,
with the proviso, that tribromo-2,4,6-tris (dihydroxy-propyl-2,3-carbamoyl)-1,3,5 benzene and tribromo-2,4,6-tris (bis-hydroxy ethyl-carbamoyl)-1,3,5 benzene are excluded,
as contrast media for the X-ray mammography

6. 2, 4, 6-tribromo-3,5-dimethoxyacetylaminobenzoic acid,
2, 4, 6-tribromo-3,5-dibromomethanesulfonylaminobenzoic acid,
2,4,6-tribromo-5-hydroxyacetylamino-N,N'-bis-(2,3-dihydroxypropyl)-N-methylisophthalamide,
2,4,6-tribromo-5-hydroxyacetylamino-N,N'-bis-(2,3-dihydroxypropyl)isophthalamide,
2,4,6-tribromo-5-hydroxyacetylamino-N-(2,3-dihydroxypropyl)-N'-(2-hydroxyethyl)isophthalamide,
2,4,6-tribromo-5-[N-methylhydroxyacetylamino]-N,N'-bis-(2,3-dihydroxypropyl)isophthalamide,
2,4,6-tribromo-5-[N-(2-hydroxyethyl)-hydroxyacetylamino]-N,N'-bis-(2,3-dihydroxypropyl)isophthalamide,
2,4,6-tribromo-5-[N-(2,3-dihydroxypropyl)hydroxyacetylamino]-N,N'-bis-(2,3-dihydroxypropyl)isophthalamide,
2,4,6-tribromo-5-bromomethanesulfonylamino-N,N'-bis-(2,3-dihydroxypropyl)-N-methyl-isophthalamide,
2,4,6-tribromo-5-hydroxyacetylamino-N-(2,3-dihydroxypropyl)-N-methylisophthalamide,
2,4,6-tribromo-5-hydroxyacetylamino-N-(1-hydroxymethyl-2,3-dihydroxypropyl)isophthalamide,
2,4,6-tribromo-5-hydroxyacetylamino-N,N,N',N'-tetrakis-(2-hydroxyethyl)isophthalamide,
2,4,6-tribromo-5-[N-(2-hydroxyethyl)-hydroxyacetylamino]-N-(1-hydroxymethyl-2,3-dihydroxypropyl)isophthalamide,
2,4,6-tribromo-5-hydroxyacetylamino-N',N'-bis-(2,3-dihydroxypropyl)-N,N'-dimethylisophthalamide,
2,4,6-tribromo-5-methoxyacetylamino-N',N'-bis-(2,3-dihydroxypropyl)-N, N'-dimethylisophthalamide,
2,4,6-tribromo-5-hydroxyacetylamino-N-(2,3-dihydroxypropyl)-N'-( 1,3-dihydroxy-2-propyl)isophthalamide,
2,4,6-tribromo-5-[N-(2-hydroxyethyl)acetylamino]-N-(1,3-dihydroxy-2-propyl)isophthalamide,
2,4,6-tribromo-5-[N-(2-hydroxyethyl)acetylamino]-N-(1-hydroxymethyl-2,3-dihydroxypropyl)isophthalamide,
N,N'-bis-[2,4,6-tribromo-3-[N-(2,3-dihydroxypropyl)-N-methylcarbamoyl]-5-[N-(2,3-dihydroxypropyl)carbamoyl]phenyl]diglycolamide,
N,N'-bis-[2,4,6-tribromo-3-[N-(2,3-dihydroxypropyl)-N-methylcarbamoyl]-5-[N-(2,3-dihydroxypropyl)carbamoyl]phenyl]malonamide,
N,N'-bis-[2,4,6-tribromo-3-[N-(2,3-dihydroxypropyl)-N-methylcarbamoyl]-5-[N-(2,3-dihydroxypropyl)carbamoyl]phenyl]-N,N'-dimethylmalonamide,
N,N'-bis-[2,4,6-tribromo-3-[N-(2,3-dihydroxypropyl)-N-methylcarbamoyl]-5-[N-(2,3-dihydroxypropyl)carbamoyl]phenyl]-N,N'-bis-(2-hydroxyethyl)diglycolamide,
N,N'-bis-[2,4,6-tribromo-3-[N-(2,3-dihydroxypropyl)-N-methylcarbamoyl]-5-[N-(2,3-dihydroxypropyl)carbamoyl]phenyl]-N,N'-bis-(2,3-dihydroxypropyl)diglycolamide,
N,N'-bis-[2,4,6-tribromo-5-carbamoyl-3-[N-(2,3-dihydroxypropyl)carbamoyl]phenyl]-N,N'-bis-(2,3-dihydroxypropyl)-malonamide,
N,N'-bis-[2,4,6-tribromo-3,5-bis-[N-(1-hydroxymethyl-2,3-dihydroxypropyl)carbamoyl]phenyl]-N,N'-dimethylmalonamide,

7. A radiological composition for use in mammography containing a brominated compound from claim 1 to 6 in a sufficient amount (10 to 400 mgBr/ml) to provide X-ray visualization together with a pharmaceutically acceptable radiological vehicle.

8. A method for x-ray visualization comprising injecting a radiological composition according to claim 7 and thereafter carrying out x-ray visualization in the energy range of mammography.
